# EUROPEAN PATENT APPLICATION

(11) **EP 4 435 095 A1**
(43) Date of publication of application: **25.09.2024**
(21) Application number: 22895980.5
(22) Date of filing: 14.11.2022
(51) Int. Cl.: C12N 5/078, C12N 5/00, C12N 5/09

(54) **IMPROVED METHOD FOR PREPARING TUMOR-INFILTRATING LYMPHOCYTE**

(30) Priority: 17.11.2021 KR 20210158291
(71) Applicant: Sungkwang Medical Foundation, Seoul 06135 (KR)
(72) Inventor: AN, Hee-Jung, Seoul 06294 (KR); KOH, A-Ra, Hwaseong-si, Gyeonggi-do 18501 (KR); JUNG, Da-Un, Seongnam-si, Gyeonggi-do 13614 (KR); KIM, Ki-Yeon, Seongnam-si, Gyeonggi-do 13372 (KR); LEE, Ji-Min, Seongnam-si, Gyeonggi-do 13489 (KR); KANG, Hyun-Gu, Hwaseong-si, Gyeonggi-do 18442 (KR)
(74) Representative: Turner, Craig Robert
(86) International application number: PCT/KR2022/017813
(87) International publication number: WO 2023/090774

(57) **Abstract**

The present invention provides an improved method for preparing tumor-infiltrating lymphocytes, the method comprising removing Treg cells that inhibit the activity of effector T cells or converting Treg cells into CD8⁺ effector T cells, whereby the tumor-infiltrating lymphocytes have excellent ability to kill tumor cells and excellent proliferative ability.

## Description

### TECHNICAL FIELD

The present invention relates to an improved method for preparing tumor-infiltrating lymphocytes. More specifically, the present invention relates to an improved method for preparing tumor-infiltrating lymphocytes, the method comprising removing Treg cells that inhibit the activity of effector T cells or converting Treg cells into effector T cells.

### BACKGROUND ART

Cancer immunotherapy refers to cancer treatments that stimulate the body's immune system and induce immune cells to attack cancer cells, unlike conventional anticancer agents that attack the cancer itself. Cancer immunotherapy is classified into immune checkpoint inhibitors, immune cell therapy, anti-cancer vaccines, and immune virus therapy/oncolytic virus therapy. Among them, immune cell therapy treats diseases by activating the body's immune response using the body's immune cells such as dendritic cells, natural killer cells, and T cells. That is, immune cell therapy refers to a therapy enhancing the body's natural healing ability, through strengthening the lowered immune function by the immune cells killing cancer cells.

Among immune cell therapy, anti-cancer immune cell therapy may be classified into tumor-infiltrating lymphocytes (TILs) prepared by proliferating the T cells infiltrated the patient's tumor tissue, T cell receptor-modified T cells (TCR-modified T cells, TCR-T) prepared by isolating T cells in the patient's blood and then introducing a gene for T cell receptor (TCR) or chimeric antigen receptor (CAR), and chimeric antigen receptor-modified T cells (CAR-modified T cells, CAR-T). Immune cell therapy includes isolating immune cells capable of killing cancer cells, cultivating and activating the isolated immune cells strongly, and returning to the body for attacking cancer cells and thus has advantages to avoid serious side effects, cancer metastasis, or recurrence that which may be caused by cytotoxicity derived from conventional cancer treatment methods. Immune cell therapy acts specifically on cancer cells to attack only cancer cells, unlike conventional chemotherapy or antibody drugs. Therefore, since immune cell therapy rarely attacks normal cells rather than cancer cells, it is expected to significantly alleviate the side effects of chemotherapy.

Tumor-infiltrating lymphocytes (TILs) show specific immune response to a tumor by isolating and using the cells having specific activity against a patient's tumor and can also exhibit immune responses in the tumor microenvironment. However, it is not easy to obtain these TILs and it is difficult to proliferate them in sufficient numbers to be applied to therapy. In addition, TILs not only contain the effector T cells (Teff cells) causing an immune response but also the regulatory T cells (Treg cells) suppressing an immune response in T cell-mediated immunity, which may lead to the reduction of efficacy of TILs.

Conventional methods for culturing TILs include isolating TILs producing interferon-gamma (IFN-γ) and then culturing them again or even include culturing non-reactive TILs. Thus, conventional methods therefor have disadvantages, such as difficulty in culturing due to the cell-isolating processes or lowering the function of TILs (J Immunother Cancer. 2018 Oct 3;6(1):102). And, since T cells are exposed to continuous antigen stimuli and inflammatory signals in cancer patients, T cells ultimately leads to exhaustion or fatigue, which results in reducing the immune cell function of T cells. Exhausted T cells progress to fatigue, due to stimulation of suppressive immune checkpoints, which results in a decrease in immune response (Nat Immunol. 2011 Jun;12(6):492-9). In order to improve these problems, it has been reported to increase the expansion of TILs by using an anti-CTLA-4 antibody as an immune checkpoint inhibitor in culturing ovarian cancer-derived TILs (Sci Rep. 2020 Mar 3;10(1):3914). In addition, it has been reported that inhibition of the PD-1/PD-L1 mechanism restores the function of fatigued T cells (Transl Cancer Res 2018;7(Suppl 4):S530-S537). However, TILs having excellent proliferative ability (expansibility) and ability to kill tumor cells, while suppressing or avoiding T cell exhaustion, have not yet been reported.

### DISCLOSURE

### Technical Problem

The present inventors carried out various studies to develop a method that can effectively prepare tumor-infiltrating lymphocytes (TILs) having excellent activity (i.e., excellent ability to kill tumor cells) and excellent proliferative ability. As the results thereof, the present inventors have found that the TILs obtained by removing Treg cells that inhibit the activity of effector T cells by selectively removing CD25⁺ cells from single cells isolated from tumor tissues or the TILs obtained by converting Treg cells into CD8⁺ effector T cells by treating single cells isolated from tumor tissues with an anti-GITR antibody not only minimize the decrease in immune response due to exhaustion but also have excellent proliferative and cancer cell killing ability.

Accordingly, it is an object of the present invention to provide a method for preparing tumor-infiltrating lymphocytes, comprising removing Treg cells by selectively removing CD25⁺ cells from single cells isolated from tumor tissues.

It is another object of the present invention to provide a method for preparing tumor-infiltrating lymphocytes, comprising converting Treg cells into CD8⁺ effector T cells by treating single cells isolated from tumor tissues with an anti-GITR antibody.

### Technical Solution

In accordance with an aspect of the present invention, there is provided a method for preparing tumor-infiltrating lymphocytes, comprising
(a) isolating single cells from a tumor tissue extracted from the human body;
(b) removing CD25⁺ cells from the single cells obtained in step (a) and then culturing the resulting cells in the presence of one or more cytokines selected from the group consisting of interleukin-2, interleukin-15, and interleukin-21; and
(c) culturing the cells obtained in step (b) in the presence of one or more cytokines selected from the group consisting of interleukin-2, interleukin-15, and interleukin-21 and peripheral blood mononuclear cells.

In the method of the present invention, the cytokine in step (b) may be interleukin-2 or a mixture of interleukin-2, interleukin-15, and interleukin-21. The culturing in step (b) may be carried out for 5 to 60 days. The culturing in step (b) may be carried out further in the presence of an anti-CD3/28 antibody. In an embodiment, the culturing in step (b) may be carried out by culturing in the presence of the cytokine and then culturing while replacing 1/3 to 2/3, e.g., about 1/2, of the medium with a medium comprising the anti-CD3/28 antibody and the cytokine every 4 to 6 days. And, the culturing may be carried out while adding the cytokine or a medium containing the cytokine every 2 to 3 days between the medium replacements every 4 to 6 days.

In the method of the present invention, the culturing in step (c) may be carried out further in the presence of phytohemagglutinin and the phytohemagglutinin may be present in an amount of 1 to 20 µg/ml. In an embodiment, the culturing in step (c) may be carried out by culturing in the presence of the cytokine, peripheral blood mononuclear cells, and optionally phytohemagglutinin and then culturing while supplementing the cytokine every 2 to 3 days from the 5th day.

In accordance with another aspect of the present invention, there is provided a method for preparing tumor-infiltrating lymphocytes, comprising
(a') isolating single cells from a tumor tissue extracted from the human body;
(b') culturing the single cells obtained in step (a') in the presence of an anti-GITR antibody and one or more cytokines selected from the group consisting of interleukin-2, interleukin-15, and interleukin-21; and
(c') culturing the cells obtained in step (b') in the presence of one or more cytokines selected from the group consisting of interleukin-2, interleukin-15, and interleukin-21 and peripheral blood mononuclear cells.

In the method of the present invention, the anti-GITR antibody may be present in an amount of 1 to 100 µg/ml. The cytokine in step (b') may be interleukin-2 or a mixture of interleukin-2, interleukin-15, and interleukin-21. The culturing in step (b') may be carried out for 5 to 60 days. The culturing in step (b') may be carried out further in the presence of an anti-CD3/28 antibody. In an embodiment, the culturing in step (b') may be carried out by culturing in the presence of the cytokine and the anti-GITR antibody and then culturing while replacing 1/3 to 2/3, e.g., about 1/2, of the medium with a medium comprising the anti-CD3/28 antibody and the cytokine every 4 to 6 days. And, the culturing may be carried out while adding the cytokine or a medium containing the cytokine every 2 to 3 days between the medium replacements every 4 to 6 days.

In the method of the present invention, the culturing in step (c') may be carried out further in the presence of phytohemagglutinin and the phytohemagglutinin may be present in an amount of 1 to 20 µg/ml. In an embodiment, the culturing in step (c') may be carried out by culturing in the presence of the cytokine, peripheral blood mononuclear cells, and optionally phytohemagglutinin and then culturing while supplementing the cytokine every 2 to 3 days from the 5th day.

### ADVANTAGEOUS EFFECTS

The tumor-infiltrating lymphocytes (TILs) obtained according to the present invention, i.e., the TILs obtained by removing Treg cells that inhibit the activity of effector T cells by selectively removing CD25⁺ cells from single cells isolated from tumor tissues or the TILs obtained by converting Treg cells into CD8⁺ effector T cells by treating single cells isolated from tumor tissues with an anti-GITR antibody not only minimize the decrease in immune response due to exhaustion but also have excellent proliferative and cancer cell killing ability. Therefore, the TILs obtained according to the present invention can be usefully applied to immune cell therapy using tumor infiltrating lymphocytes.

### DESCRIPTION OF DRAWINGS

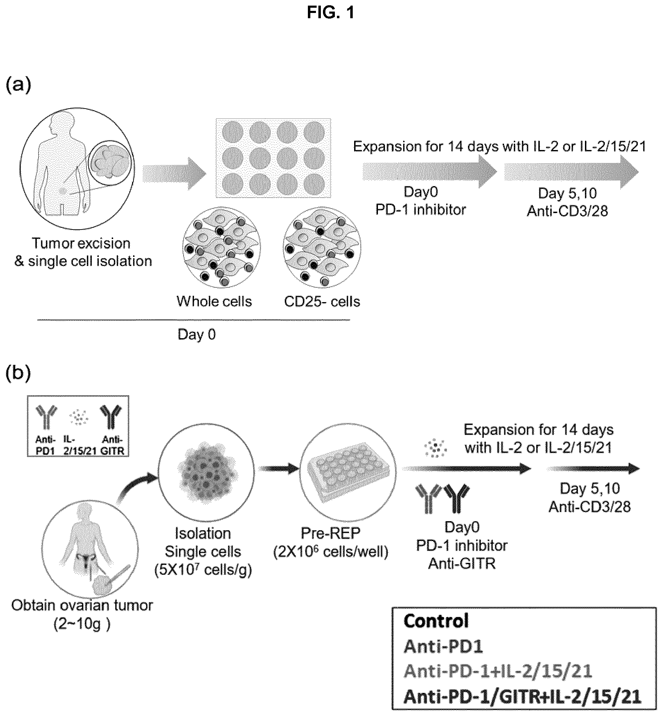

FIG. 1 is a schematic diagram showing an example of the pre-REP culture of rapid expansion protocol (REP) in the method for preparing tumor-infiltrating lymphocytes according to the present invention. FIG. 1a is a schematic diagram showing an example of the pre-REP culture of TILs, which includes removing Treg cells that inhibit the activity of effector T cells by selectively removing CD25⁺ cells. FIG. 1b is a schematic diagram showing an example of the pre-REP culture of TILs, which includes converting Treg cells into CD8⁺ effector T cells by treating with an anti-GITR antibody.
FIG. 2 shows the number of proliferated cells (FIG. 2a) and the proliferation fold (FIG. 2b) of the TILs obtained with or without treatment with a PD-1 inhibitor.
FIG. 3 shows the results obtained by evaluating the cancer cell killing ability of the TILs obtained with or without treatment with a PD-1 inhibitor.
FIG. 4 shows the number of proliferated cells (FIG. 4a) and the proliferation fold (FIG. 4b) of the TILs obtained after removing CD25⁺ cells or the TILs obtained without removing CD25⁺ cells.
FIG. 5 shows the results obtained by measuring the contents of lymphocytes (FIG. 5a), the ratio of CD4⁺, CD8⁺, CD4⁺CD8⁺, and CD4-CD8- cells in the lymphocytes (FIG. 5b), and the degree of T cell differentiation (FIG. 5c), in the TILs obtained after removing CD25⁺ cells or the TILs obtained without removing CD25⁺ cells.
FIG. 6 shows the results obtained by measuring the expressions of CD137 and PD-1 in CD4⁺ cells (FIG. 6a) and in CD8⁺ cells (FIG. 6a), in the TILs obtained after removing CD25⁺ cells or the TILs obtained without removing CD25⁺ cells.
FIG. 7 shows the results obtained by measuring the cytotoxicity against cancer cells (FIG. 7a) and the IFN-γ secretion (FIG. 7b) of the TILs obtained after removing CD25⁺ cells or the TILs obtained without removing CD25⁺ cells.
FIG. 8 shows the results obtained by measuring the secretion of IFN-γ from the TILs obtained after removing CD25⁺ cells or the TILs obtained without removing CD25⁺ cells.
FIG. 9 shows the cytotoxicity against cancer cells of the TILs obtained by carrying out REP on the TILs obtained after removing CD25⁺ cells or on the TILs obtained using an anti-GITR-antibody without removing CD25⁺ cells.
FIG. 10 shows the proliferation pattern (FIG. 10a), the number of proliferated cells (FIG. 10b), and the proliferation fold (FIG. 10c) of the TILs obtained without using an anti-GITR-antibody or the TILs obtained using an anti-GITR-antibody.
FIG. 11 shows the results obtained by measuring the ratios of CD8⁺ cells (FIG. 11a), CD4⁺ cells (FIG. 11b), CD4⁺Foxp3⁺ cells (FIG. 11c) in CD3⁺ lymphocytes, in the TILs obtained without using an anti-GITR-antibody or the TILs obtained using an anti-GITR-antibody.
FIG. 12 shows the results obtained by measuring expressions of CD137 and Granzyme B (FIG. 12a) and Granzyme B (FIG. 12b) of CD8⁺ cells, in the TILs obtained without using an anti-GITR-antibody or the TILs obtained using an anti-GITR-antibody.
FIG. 13 shows the results obtained by evaluating the cytotoxicity against cancer cells by FACs (FIG. 13a) or microscopic observation (FIG. 13b) of the TILs obtained without using an anti-GITR-antibody or the TILs obtained using an anti-GITR-antibody; and the results obtained by measuring IFN-γ secretion therefrom (FIG. 13c).
FIG. 14 shows the number of proliferated cells (FIG. 14a), the proliferation fold (FIG. 14b), and the cell number (FIG. 14c), after carrying out the REP using PHA for the TILs obtained without using an anti-GITR-antibody or the TILs obtained using an anti-GITR-antibody.
FIG. 15 shows the results obtained by measuring the ratios of CD8⁺ cells (FIG. 15a), CD4⁺ cells (FIG. 15b), CD4⁺Foxp3⁺ cells (FIG. 15c) in CD3⁺ lymphocytes, in the TILs obtained after carrying out the REP using PHA for the TILs obtained without using an anti-GITR-antibody or the TILs obtained using an anti-GITR-antibody.
FIG. 16 shows the results obtained by measuring expressions of CD137 (FIG. 16a), OX40 (FIG. 16b), Granzyme B (FIG. 16c) of CD8⁺ cells, in the TILs obtained after carrying out the REP using PHA for the TILs obtained without using an anti-GITR-antibody or the TILs obtained using an anti-GITR-antibody.
FIG. 17 shows the results obtained by evaluating the cytotoxicity against cancer cells by microscopic observation (FIG. 17a) of the TILs obtained after carrying out the REP using PHA for the TILs obtained without using an anti-GITR-antibody or the TILs obtained using an anti-GITR-antibody; and the results obtained by measuring IFN-γ secretion therefrom (FIG. 17b).

### BEST MODE FOR CARRYING OUT THE INVENTION

The present invention provides a method for preparing tumor-infiltrating lymphocytes (TILs), which can not only minimize the decrease in immune response due to exhaustion but also have excellent proliferative and cancer cell killing ability.

In an aspect, the present invention provides a method for preparing tumor-infiltrating lymphocytes, comprising
(a) isolating single cells from a tumor tissue extracted from the human body;
(b) removing CD25⁺ cells from the single cells obtained in step (a) and then culturing the resulting cells in the presence of one or more cytokines selected from the group consisting of interleukin-2, interleukin-15, and interleukin-21; and
(c) culturing the cells obtained in step (b) in the presence of one or more cytokines selected from the group consisting of interleukin-2, interleukin-15, and interleukin-21 and peripheral blood mononuclear cells.

The method of the present invention includes isolating single cells from a tumor tissue extracted from the human body [i.e., step (a)]. The tumor tissue may be derived from various cancer patients. For example, the tumor tissue may be derived from the tissue extracted from a patient with solid cancer. That is, the tumor tissue may be derived from any solid cancer tissue having a similar cancer microenvironment. In one embodiment, the tumor tissue may be an ovarian cancer tissue isolated from an ovarian cancer patient, but not limited thereto. The isolation of single cells from a tumor tissue may be carried out according to conventional methods used in the art (e.g., Cytotherapy 2019 March; 21(3): 307-314, etc.). For example, the isolated tumor tissue may be sectioned into 1-3 mm³ fragments and then single cells may be isolated therefrom using a device such as the gentleMACS Dissociator.

The step (b) of method of the present invention includes removing CD25⁺ cells from the single cells. Removal of CD25⁺ cells can be carried out by treating with commercially available CD25 microbeads and then using, e.g., MACS separator equipment. The treating with CD25 microbeads may be carried out for example, by adding 20 µl of CD25 microbeads per 1 × 10⁷ cells, followed by incubating at about 4°C for 10 to 30 minutes, preferably for about 15 to 20 minutes. The cells obtained by using MACS separator equipment, etc. (i.e., CD25⁻ cells) can be stored/maintained by washing and diluting with a medium such as RPMI 1640 GlutaMAX medium.

The step (b) of method of the present invention also includes culturing the cells obtained above (i.e., CD25⁻ cells) in the presence of one or more cytokines selected from the group consisting of interleukin-2, interleukin-15, and interleukin-21 (i.e., pre-REP culture step).

The culturing in the step (b) may be carried out in the presence or absence of a PD-1 (programmed cell death-1) inhibitor. When the culturing in the step (b) is carried out in the presence of a PD-1 inhibitor, the PD-1 inhibitor includes a known antibody or a chemical substance (a synthetic compound). For example, the PD-1 inhibitor may be an anti-PD-1 antibody such as J116 monoclonal antibody (BioXcell), but not limited thereto. The PD-1 inhibitor may be used in an amount of 1 to 100 µg/ml, preferably about 10 µg/ml, but not limited thereto.

In the culturing in the step (b), the cytokine may be interleukin-2 (IL-2). The IL-2 may be used in an amount of 10 to 6,000 U/ml, preferably about 3,000 U/ml, but not limited thereto. In addition, the cytokine may be a mixture of interleukin-2 (IL-2), interleukin-15 (IL-15), and interleukin-21 (IL-21). For example, the cytokine may be a mixture of 10 to 6,000 U/ml of IL-2, 20 to 300 U/ml of IL-15, and 5 to 100 U/ml of IL-21, preferably a mixture of 3,000 U/ml of IL-2, 180 U/ml of IL-15, and 40 U/ml of IL-21, but not limited thereto.

The culturing in the step (b) may be carried out for 5 to 60 days, preferably for 10 to 50 days, more preferably for 12 to 30 days, particularly preferably for about 14 days. The culture medium may be any conventional medium used in the field of the preparation of tumor-infiltrating lymphocytes. For example, the culture medium may be a culture medium obtained by mixing the RPMI 1640 GlutaMAX medium supplemented with human AB serum, penicillin-streptomycin, 2-mercaptoethanol, etc. and an AIM-V serum-free medium in a 1:1 ratio, but not limited to thereto.

In the culturing in the step (b), the cytokine (IL-2 or IL-2/15/21) and optionally a PD-1 inhibitor are added to the medium on Day 0 of culture. For proliferation and activation of lymphocytes, an anti-CD3/28 antibody may be added thereto after 4 to 6 days, for example, after about 5 days, and then supplemented every 4 to 6 days, for example, every 5 days. The amount of the anti-CD3/28 antibody added is not limited and may be used in an amount of, for example, 5 to 50 µg/ml, preferably 10 to 25 µg/ml. And, the cytokine (IL-2 or IL-2/15/21) may be supplemented every 2 to 3 days, for example, about every 2 days. Accordingly, in an embodiment, the culturing may be carried out by culturing in the presence of the cytokine (IL-2 or IL-2/15/21) and optionally a PD-1 inhibitor and then culturing while replacing 1/3 to 2/3, e.g., 1/2, of the medium with a medium comprising an anti-CD3/28 antibody and the cytokine (IL-2 or IL-2/15/21) every 4 to 6 days. And, the culturing may be carried out while adding the cytokine or a medium containing the cytokine every 2 to 3 days between the medium replacements every 4 to 6 days. In said embodiment, the amount of IL-2 or IL-2/15/21 used is as described above.

The method of the present invention includes culturing the cells obtained in step (b) in the presence of one or more cytokines selected from the group consisting of interleukin-2, interleukin-15, and interleukin-21 and peripheral blood mononuclear cells (PBMCs) [i.e., step (c)].

The step (c) may be carried out, using the cells obtained in the step (b), according to the Rapid Expansion Protocol (REP) method according to the conventional methods for the preparation of TILs. That is, step (c) may be carried out by culturing the cells obtained in the step (b) in the presence of cytokine (IL-2 or IL-2/15/21) and PBMCs.

It has been found by the present invention that, when the Rapid Expansion Protocol (REP) is carried out by additionally adding phytohemagglutinin (PHA), proliferation and activity of the TILs are further increased, compared to when using an anti-CD3 antibody. Therefore, the culturing in step (c) may be carried out by culturing the cells obtained in the step (b) further in the presence of phytohemagglutinin. The phytohemagglutinin may be present in an amount of 1 to 20 µg/ml, but is limited thereto.

The culturing in the step (c) may be carried out for 5 to 60 days, preferably for 10 to 50 days, more preferably for 12 to 30 days, particularly preferably for about 14 days. The culture medium may be any conventional medium used in the field of the preparation of tumor-infiltrating lymphocytes. For example, the culture medium may be a culture medium obtained by mixing the RPMI 1640 GlutaMAX medium supplemented with human AB serum, penicillin-streptomycin, 2-mercaptoethanol, etc. and an AIM-V serum-free medium in a 1:1 ratio, but not limited to thereto.

In the culturing in the step (c), the cytokine (IL-2 or IL-2/15/21), PBMCs (e.g., 55Gy irradiated PBMCs), and optionally phytohemagglutinin are added to the medium on Day 0 of the Rapid Expansion Protocol culture. The cytokine (IL-2 or IL-2/15/21) may be supplemented every 2 to 3 days, for example, about every 2 days, from Day 5 of the Rapid Expansion Protocol culture. In an embodiment, the culturing in step (c) may be carried out by culturing in the presence of the cytokine, peripheral blood mononuclear cells, and optionally phytohemagglutinin and then culturing while supplementing the cytokine (IL-2 or IL-2/15/21) every 2 to 3 days from the 5th day. In said embodiment, the amount of IL-2 or IL-2/15/21 used is as described above with respect to the pre-REP culture.

The cells obtained by culturing as described above, that is, tumor-infiltrating lymphocytes, may be isolated from the culture medium according to conventional methods. For example, the TILs may be harvested by centrifuging at about 1,500 rpm at 4°C for 5 minutes to remove the supernatant. The harvested TILs may be usefully applied for immune cell therapy.

In another aspect, the present invention provides a method for preparing tumor-infiltrating lymphocytes, comprising
(a') isolating single cells from a tumor tissue extracted from the human body;
(b') culturing the single cells obtained in step (a') in the presence of an anti-GITR antibody and one or more cytokines selected from the group consisting of interleukin-2, interleukin-15, and interleukin-21; and
(c') culturing the cells obtained in step (b') in the presence of one or more cytokines selected from the group consisting of interleukin-2, interleukin-15, and interleukin-21 and peripheral blood mononuclear cells.

The method of the present invention includes isolating single cells from a tumor tissue isolated from the human body [i.e., step (a')]. The step (a') may be carried out in the same manner as the step (a).

The method of the present invention includes culturing the single cells obtained in step (a') in the presence of an anti-GITR antibody and one or more cytokines selected from the group consisting of interleukin-2, interleukin-15, and interleukin-21 (pre-REP culture step) [i.e., step (b')].

In the step (b') of the method of the present invention, the anti-GITR antibody includes a known antibody or a chemical substance (a synthetic compound). The anti-GITR antibody may be used in an amount of 1 to 100 µg/ml, preferably about 20 µg/ml, but not limited thereto.

The culturing in the step (b') may be carried out in the presence or absence of a PD-1 (programmed cell death-1) inhibitor. When the culturing in the step (b') is carried out in the presence of a PD-1 inhibitor, the PD-1 inhibitor includes a known antibody or a chemical substance (a synthetic compound). For example, the PD-1 inhibitor may be an anti-PD-1 antibody such as J116 monoclonal antibody (BioXcell), but not limited thereto. The PD-1 inhibitor may be used in an amount of 1 to 100 µg/ml, preferably about 10 µg/ml, but not limited thereto.

In the culturing in the step (b'), the cytokine may be interleukin-2 (IL-2). The IL-2 may be used in an amount of 10 to 6,000 U/ml, preferably about 3,000 U/ml, but not limited thereto. In addition, the cytokine may be a mixture of interleukin-2 (IL-2), interleukin-15 (IL-15), and interleukin-21 (IL-21). For example, the cytokine may be a mixture of 10 to 6,000 U/ml of IL-2, 20 to 300 U/ml of IL-15, and 5 to 100 U/ml of IL-21, preferably a mixture of 3,000 U/ml of IL-2, 180 U/ml of IL-15, and 40 U/ml of IL-21, but not limited thereto.

The culturing in the step (b') may be carried out for 5 to 60 days, preferably for 10 to 50 days, more preferably for 12 to 30 days, particularly preferably for about 14 days. The culture medium may be any conventional medium used in the field of the preparation of tumor-infiltrating lymphocytes. For example, the culture medium may be a culture medium obtained by mixing the RPMI 1640 GlutaMAX medium supplemented with human AB serum, penicillin-streptomycin, 2-mercaptoethanol, etc. and an AIM-V serum-free medium in a 1:1 ratio, but not limited to thereto.

In the culturing in the step (b'), the cytokine (IL-2 or IL-2/15/21), the anti-GITR antibody, and optionally a PD-1 inhibitor are added to the medium on Day 0 of culture. For proliferation and activation of lymphocytes, an anti-CD3/28 antibody may be added thereto after 4 to 6 days, for example, after about 5 days, and then supplemented every 4 to 6 days, for example, every about 5 days. The amount of the anti-CD3/28 antibody added is not limited and may be used in an amount of, for example, 5 to 50 µg/ml, preferably 10 to 25 µg/ml. And, the cytokine (IL-2 or IL-2/15/21) may be supplemented every 2 to 3 days, for example, about every 2 days. Accordingly, in an embodiment, the culturing may be carried out by culturing in the presence of the cytokine (IL-2 or IL-2/15/21), the anti-GITR antibody, and optionally a PD-1 inhibitor and then culturing while replacing 1/3 to 2/3, e.g., 1/2, of the medium with a medium comprising the anti-CD3/28 antibody and the cytokine (IL-2 or IL-2/15/21) every 4 to 6 days. And, the culturing may be carried out while adding the cytokine or a medium containing the cytokine every 2 to 3 days between the medium replacements every 4 to 6 days. In said embodiment, the amount of IL-2 or IL-2/15/21 used is as described above.

The method of the present invention includes culturing the cells obtained in step (b') in the presence of one or more cytokines selected from the group consisting of interleukin-2, interleukin-15, and interleukin-21 and peripheral blood mononuclear cells [i.e., step (c')].

The step (c') may be carried out, using the cells obtained in the step (b'), according to the Rapid Expansion Protocol (REP) method according to the conventional methods for the preparation of TILs. That is, step (c') may be carried out by culturing the cells obtained in the step (b') in the presence of cytokine (IL-2 or IL-2/15/21) and PBMCs.

It has been found by the present invention that, when the Rapid Expansion Protocol (REP) is carried out by additionally adding phytohemagglutinin (PHA), proliferation and activity of the TILs are further increased, compared to when using an anti-CD3 antibody. Therefore, the culturing in step (c') may be carried out by culturing the cells obtained in the step (b') further in the presence of phytohemagglutinin. The phytohemagglutinin may be present in an amount of 1 to 20 µg/ml, but is limited thereto.

The culturing in the step (c') may be carried out for 5 to 60 days, preferably for 10 to 50 days, more preferably for 12 to 30 days, particularly preferably for about 14 days. The culture medium may be any conventional medium used in the field of the preparation of tumor-infiltrating lymphocytes. For example, the culture medium may be a culture medium obtained by mixing the RPMI 1640 GlutaMAX medium supplemented with human AB serum, penicillin-streptomycin, 2-mercaptoethanol, etc. and an AIM-V serum-free medium in a 1:1 ratio, but not limited to thereto.

In the culturing in the step (c'), the cytokine (IL-2 or IL-2/15/21), PBMCs (e.g., 55Gy irradiated PBMCs), and optionally phytohemagglutinin are added to the medium on Day 0 of the Rapid Expansion Protocol culture. The cytokine (IL-2 or IL-2/15/21) may be supplemented every 2 to 3 days, for example, about every 2 days, from Day 5 of the Rapid Expansion Protocol culture. In an embodiment, the culturing in step (c') may be carried out by culturing in the presence of the cytokine, peripheral blood mononuclear cells, and optionally phytohemagglutinin and then culturing while supplementing the cytokine (IL-2 or IL-2/15/21) every 2 to 3 days from the 5th day. In said embodiment, the amount of IL-2 or IL-2/15/21 used is as described above with respect to the pre-REP culture.

The cells obtained by culturing as described above, that is, tumor-infiltrating lymphocytes, may be isolated from the culture medium according to conventional methods. For example, the TILs may be harvested by centrifuging at about 1,500 rpm at 4°C for 5 minutes to remove the supernatant. The harvested TILs may be usefully applied for immune cell therapy.

Hereinafter, the present invention will be described more specifically by the following examples and test examples. However, the following examples and test examples are provided only for illustrations and thus the present invention is not limited to or by them.

### Example 1: Preparation of tumor-infiltrating lymphocytes (TILs)

Tumor tissues were obtained from ovarian cancer patients. Written informed consent was obtained from the patients before participating in this study. This study was approved by the institutional review board of CHA Bundang Medical Center.

### (1) Isolation of single cells from tumor tissues

The tumor tissues isolated from the body were sectioned into 1-3 mm³ fragments and then single cells were isolated from the sectioned tumor tissues using a gentleMACS Dissociator. The isolated single cells were maintained in the culture medium obtained by mixing the RPMI 1640 GlutaMAX medium shown in Table 1 (Thermo Scientific, 72400-047) and the AIM-V serum-free medium (Gibco, 12055-091) in a 1:1 ratio.

**Table 1**

| RPMI 1640 GlutaMAX medium | | | |
|---|---|---|---|
| Ingredient | Supplier | Cat no. | Final concentration |
| Human AB Serum | Merck | H4522 | 10% |
| Penicillin-Streptomycin | Gibco | 15140122 | 1% |
| 2- Mercaptoethanol | Gibco | 21985-023 | 0.1% |

### (2) Removal of CD25⁺ cells

The single cells isolated/maintained in (1) above were centrifuged at 1,500 rpm for 3 minutes and then the supernatant was removed therefrom. The resulting cells were washed with 2 mL of MACS buffer. The MACS buffer was prepared by adding 0.5% bovine serum albumin (BSA) to phosphate buffered saline (PBS). The washed cell solution was centrifuged again at 1,500 rpm for 3 minutes and then the supernatant was removed. 20 µl of the CD25 MicroBead II solution (Miltenyi Biotec, 130-092-983) and 80 µl of the MACS buffer were added per 1 × 10⁷ cells, followed by incubating at 4°C for 15 minutes. After the incubation, the cells were washed with about 2 ml of the MACS buffer, centrifuged at 1,500 rpm for 3 minutes, and the resulting pellets were diluted in 500 µl of the MACS buffer. The CD25⁺ cells were removed therefrom using a MACS column (LS column) and a MACS separator (MidiMACS) according to the manufacturer's instructions (CD25 MicroBeads II, Miltenyi Biotec, 130-092-983). After centrifuging at 1,500 rpm for 3 minutes, the supernatant was removed. To the resulting cells (CD25⁻ cells), was added the culture medium obtained by mixing the RPMI 1640 GlutaMAX medium (Thermo Scientific, 72400-047) and the AIM-V serum-free medium (Gibco, 12055-091) in a 1:1 ratio, to prepare 1X10⁶ cells/2ml.

### (3) Cell culture (pre-REP)

The CD25⁻ cells obtained in (2) above were cultured for 30 days in the culture medium supplemented with 10 µg/ml of an anti-CD3/28 antibody (stem cell, 10990), 3,000 U/ml of IL-2 (PEPROTECH, 200-02), and 10 µg/ml of a PD-1 inhibitor (J116 monoclonal antibody, BioXcell).

IL-2 and the PD-1 inhibitor were added to the culture medium on Day 0 of culture. Every 5 days after the start of culture, the culturing was carried out by replacing approximately 1/2 of the culture medium with a medium supplemented with the anti-CD3/28 antibody and IL-2. In addition, the culturing was carried out while adding the culture medium supplemented with IL-2 every 2 days between the medium replacements every 5 days. Specifically, on Day 0 of culture, the CD25⁻ cells obtained in (2) above (1×10⁶ cells/2ml) were added to a G-rex 24-well plate (wilsonwolf, 80192M) and then cultured in 2 ml of the culture medium supplemented with 3,000 U/ml of IL-2 (PEPROTECH, 200-02) and 10 µg/ml of the PD-1 inhibitor (J116 monoclonal antibody, BioXcell). After 5 days (on the 5th day of pre-REP culture), 1 ml of the culture medium (i.e., the half thereof) was removed. 1 ml of the culture medium supplemented with 10 µg/ml of the anti-CD3/28 antibody (stem cell, 10990) and 3,000 U/ml of IL-2 was added thereto (i.e., 2 ml in total) and then cultured. After 2 days (on the 7th day of pre-REP culture), 1 ml of the culture medium (i.e., the half thereof) was removed. 1 ml of the culture medium supplemented with 3,000 U/ml of IL-2 was added (total 2 ml) and cultured. After 3 days (on the 10th day of pre-REP culture), 1 ml of the culture medium (i.e., the half thereof) was removed. 1 ml of the culture medium supplemented with 10 µg/ml of the anti-CD3/28 antibody (stem cell, 10990) and 3,000 U/ml of IL-2 was added thereto (i.e., 2 ml in total) and then cultured. After 2 days (on the 12th day of pre-REP culture), 1 ml of the culture medium (i.e., the half thereof) was removed. 1 ml of the culture medium supplemented with 3,000 U/ml of IL-2 was added thereto (i.e., 2 ml in total) and then cultured for 2 days. That is, the culturing was carried out for 14 days in total (i.e., pre-REP culture for 14 days in total). The pre-REP culture after 14 days was performed in the same manner as the pre-REP culture for 14 days. The TILs obtained by the pre-REP culture for 14 days as described above are referred to as "CD25⁻ + IL2" in the test examples below.

### (4) Rapid Expansion Protocol (REP)

5 ml of the culture medium supplemented with 2 µg/ml of phytohemagglutinin (PHA) and 3,000 U/ml of IL-2 (PEPROTECH, 200-02) was added to a T25 Flask and the TILs obtained in (3), i.e. "CD25- + IL2" (2×10⁵ cells) and peripheral mononuclear cells (irradiated with 55Gy, 2×10⁷ cells) were co-cultured therein. After 5 days (on the 5th day of REP culture), 5 ml of the culture medium supplemented with 3,000 U/ml of IL-2 was added thereto (i.e., 10 ml in total) and then cultured. After 2 days (on the 7th day of REP culture), 20 ml of the culture medium supplemented with 3,000 U/ml of IL-2 was added thereto (i.e., 30 ml in total) and then cultured. After 3 days (on the 10th day of REP culture), the cells were collected in a 50ml conical tube and centrifuged at 1,200 rpm for 3 minutes. After removing 15 ml of the culture medium (i.e., the half thereof), 15 ml of the culture medium supplemented with 3,000 U/ml of IL-2 was added thereto (i.e., 30 ml in total) and then cultured. After 2 days (on the 12th day of REP culture), 60 ml of the culture medium supplemented with 3,000 U/ml of IL-2 was added thereto (i.e., 90 ml in total) and then cultured for 2 days. That is, the culturing was carried out for 14 days in total (i.e., REP culture for 14 days in total).

### Example 2: Preparation of tumor-infiltrating lymphocytes (TILs)

### (1) Cell culture (pre-REP)

The TILs were prepared by culturing according to the same manner as in (3) of Example 1 for 30 days in total, in the culture medium supplemented with 10 µg/ml of an anti-CD3/28 antibody (stem cell, 10990), 3,000 U/ml of IL-2 (PEPROTECH, 200-02), 180 U/ml of IL-15 (PEPROTECH, 200-02), 40 U/ml of IL-21 (PEPROTECH, 200-21), and 10 µg/ml of a PD-1 inhibitor (J116 monoclonal antibody, BioXcell).

IL-2/IL-15/IL-21 and the PD-1 inhibitor were added to the culture medium on Day 0 of culture. Every 5 days after the start of culture, the culturing was carried out by replacing approximately 1/2 of the culture medium with a medium supplemented with the anti-CD3/28 antibody and IL-2/IL-15/IL-21. In addition, the culturing was carried out while adding the culture medium supplemented with IL-2/IL-15/IL-21 every 2 days between the medium replacements every 5 days. Specifically, on Day 0 of culture, the cells obtained in the above (1×10⁶ cells/2ml) were added to a G-rex 24-well plate (wilsonwolf, 80192M) and then cultured in 2 ml of the culture medium supplemented with 3,000 U/ml of IL-2 (PEPROTECH, 200-02), 180 U/ml of IL-15 (PEPROTECH, 200-02), 40 U/ml of IL-21 (PEPROTECH, 200-21), and 10 µg/ml of the PD-1 inhibitor (J116 monoclonal antibody, BioXcell). After 5 days (on the 5th day of pre-REP culture), 1 ml of the culture medium (i.e., the half thereof) was removed. 1 ml of the culture medium supplemented with 10 µg/ml of the anti-CD3/28 antibody (stem cell, 10990), 3,000 U/ml of IL-2 (PEPROTECH, 200-02), 180 U/ml of IL-15 (PEPROTECH, 200-02), and 40 U/ml of IL-21 (PEPROTECH, 200-21) was added thereto (i.e., 2 ml in total) and then cultured. After 2 days (on the 7th day of pre-REP culture), 1 ml of the culture medium (i.e., the half thereof) was removed. 1 ml of the culture medium supplemented with 3,000 U/ml of IL-2, 180 U/ml of IL-15 (PEPROTECH, 200-02), and 40 U/ml of IL-21 (PEPROTECH, 200-21) was added (total 2 ml) and cultured. After 3 days (on the 10th day of pre-REP culture), 1 ml of the culture medium (i.e., the half thereof) was removed. 1 ml of the culture medium supplemented with 10 µg/ml of the anti-CD3/28 antibody (stem cell, 10990), 3,000 U/ml of IL-2 (PEPROTECH, 200-02), 180 U/ml of IL-15 (PEPROTECH, 200-02), and 40 U/ml of IL-21 (PEPROTECH, 200-21) was added thereto (i.e., 2 ml in total) and then cultured. After 2 days (on the 12th day of pre-REP culture), 1 ml of the culture medium (i.e., the half thereof) was removed. 1 ml of the culture medium supplemented with 3,000 U/ml of IL-2, 180 U/ml of IL-15 (PEPROTECH, 200-02), and 40 U/ml of IL-21 (PEPROTECH, 200-21) was added thereto (i.e., 2 ml in total) and then cultured for 2 days. That is, the culturing was carried out for 14 days in total (i.e., pre-REP culture for 14 days in total). The pre-REP culture after 14 days was performed in the same manner as the pre-REP culture for 14 days. The TILs obtained by the pre-REP culture for 14 days as described above are referred to as "CD25⁻ + IL2/15/21" in the test examples below.

### (2) Rapid Expansion Protocol (REP)

REP culture for the CD25⁻ + IL2/15/21 was carried out by culturing according to the same manner as in (4) of Example 1 for 14 days in total, except for using 3,000 U/ml of IL-2 (PEPROTECH, 200-02), 180 U/ml of IL-15 (PEPROTECH, 200-02), and 40 U/ml of IL-21 (PEPROTECH, 200-21) as cytokines.

### Comparative Example 1: Preparation of tumor-infiltrating lymphocytes (TILs)

### (1) Preparation of Whole + IL2 TILs

TILs were prepared without performing the process (2) of Example 1 (CD25⁺ cell removal process). That is, a 14-day pre-REP culture was carried out according to the same manner as in (3) of Example 1, except for adding the single cells isolated from tumor tissue (1×10⁶ cells/2ml) to a G-rex 24-well plate (wilsonwolf, 80192M) on Day 0 of culture. The obtained TILs are referred to as "Whole + IL2" in the test examples below.

### (2) Preparation of Whole + IL2/15/21 TILs

TILs were prepared without performing the process (2) of Example 1 (CD25⁺ cell removal process). That is, a 14-day pre-REP culture was carried out according to the same manner as in Example 2, except for adding the single cells isolated from tumor tissue (1×10⁶ cells/2ml) to a G-rex 24-well plate (wilsonwolf, 80192M) on Day 0 of culture and using 3,000 U/ml of IL-2 (PEPROTECH, 200-02), 180 U/ml of IL-15 (PEPROTECH, 200-02), and 40 U/ml of IL-21 (PEPROTECH, 200-21) as cytokines. The obtained TILs are referred to as "Whole + IL2/15/21" in the test examples below.

### Test Example 1: Evaluation on proliferation of TILs according to PD-1 inhibitor treatment

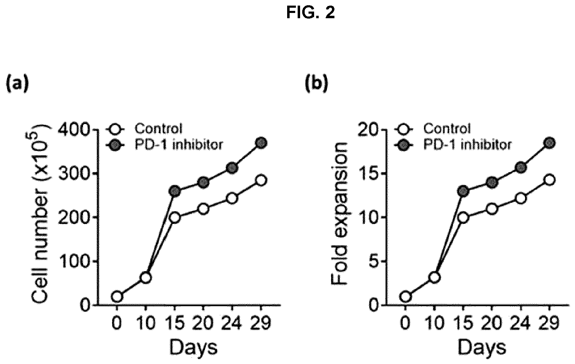

While the TILs were grown for 30 days according to Example 1, the number of proliferated cells was measured (test group). In addition, while the TILs were grown for 30 days according to the same manner as in Example 1 except that the PD-1 inhibitor was not added in (3) of Example 1, the number of proliferated cells was measured (control group). The number of cells was measured with a LUNA cell counter after staining with Acridine Orange/Propidium Iodide staining reagent (logos bio, F23001). The results are as shown in FIG. 2. As can be seen from the results of FIG 2, the control group and the test group (i.e., the proliferation group of Example 1) were proliferated from 1X10⁶ cells on Day 0 of culture to 2.85×10⁷ cells (14.3 times) and 3.7X10⁷ cells (18.5 times) on the 14th day of culture, respectively.

### Test Example 2: Evaluation on tumor cell-killing ability of TILs according to PD-1 inhibitor treatment

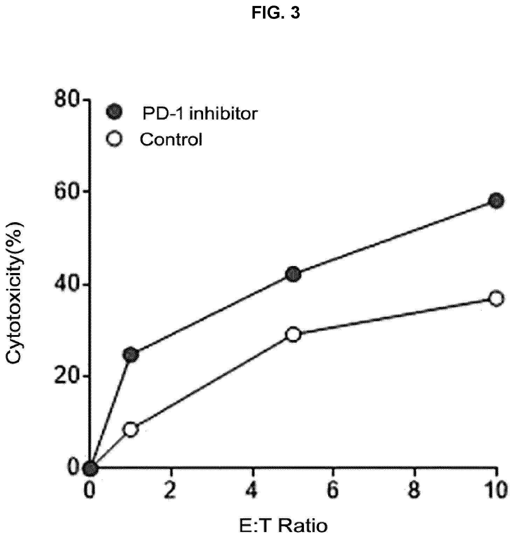

The tumor cell-killing abilities of the TILs obtained by pre-REP culture for 14 days according to Example 1 (test group); and the TILs obtained by pre-REP culture for 14 days according to the same manner as in Example 1 except that the PD-1 inhibitor was not added in (3) of Example 1 (control group) were evaluated using a CFSE/7AAD method. Specifically, cancer cells from the tumor tissue of an ovarian cancer patient were diluted in PBS to 2×10⁵ cells/ml and then fluorescently stained with 5 µM of CFSE (Carboxyfluorescein diacetate succinimidyl ester, Invitrogen, C34570). After separating the stained cells by centrifugation at 2000 rpm for about 3 minutes, the stained cancer cells were added in the concentration of 2×10⁵ cells/ml to the cancer cell culture medium [obtained by mixing the RPMI 1640 GlutaMAX medium (Thermo Scientific, 72400-047) supplemented with the excipients of Table 1 above and the AIM-V serum-free medium (Gibco, 12055-091) in a 1:1 ratio] and then 100 µl thereof was added into each well of a v-bottom 96-well plate. The TILs of the test group and the control group were diluted in the cancer cell culture medium to 2×10⁶ cells/ml, respectively, and then added into each well at a cancer cell:TILs ratio of 1:1, 1:5, and 1:10. The amount of culture medium containing cells per well was set to 200 µl. After culturing at 37°C and 5% CO₂ conditions for 4 hours, the cell mixture was pelleted to remove the culture medium and then stained for 15 minutes at room temperature with 7-AAD (7-amino-actinomycin D, Thermo, A1310) for staining dead cells. The stained mixture was washed once with phosphate-buffered saline (PBS) and then suspended in a FACS buffer. The percentage (%) of 7AAD+ cells in CFSE+ cells was measured using a CytoFLEX flow cytometry (Beckman Coulter) and analyzed with a FlowJo version 10.1 software (Treestar inc, Ashland). The results are as shown in FIG. 3.

### Test Example 3: Evaluation on proliferation of TILs according to CD25⁺ cell removal

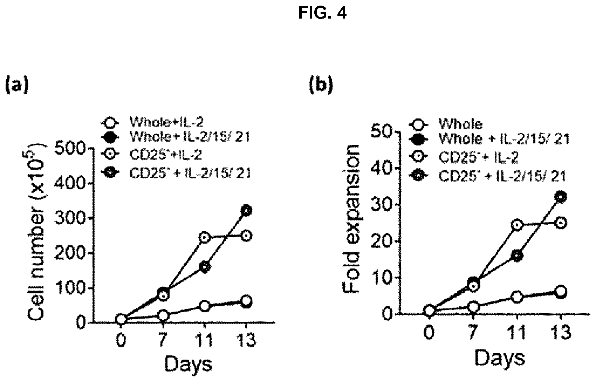

While the CD25⁻ + IL2, CD25⁻ + IL2/15/21, Whole + IL2, and Whole + IL2/15/21 were grown for 14 days, the number of proliferated cells was measured. The number of cells was measured with a LUNA cell counter after staining with Acridine Orange/Propidium Iodide staining reagent (logos bio, F23001). The results are as shown in FIG. 4. As can be seen from the results of FIG 4, the TILs containing CD25⁺ cells were proliferated from 1X10⁶ cells on Day 0 of culture to 6.42×10⁶ cells (Whole + IL-2, 6.4 times) and 5.85×10⁶ cells (Whole + IL-2/15/21, 5.9 times) on the 14th day of culture. The TILs cultured after removing CD25⁺ cells were proliferated from 1×10⁶ cells on Day 0 of culture to 2.5×10⁷ cells (CD25- + IL-2, 25.1 times) and 3.22×10⁷ cells (CD25⁻ + IL-2/15/21, 32.2 times) on the 14th day of culture. Therefore, it can be confirmed from the above results that, when CD25⁺ cells were removed and then the culture of TILs was carried out in various cytokines, the proliferation fold thereof was increased to 3.9 times (IL-2 culture group) or 5.4 times (IL-2/15/21 culture group), compared to those of the TILs containing CD25⁺ cells.

### Test Example 4: Evaluation on differentiation and activity of the immune cells

The cell culture of CD25⁻ + IL2, the cell culture of CD25⁻ + IL2/15/21, the cell culture of Whole + IL2, and the cell culture of Whole + IL2/15/21 were respectively centrifuged at 1,500 rpm for 5 minutes at 4°C to remove each supernatant and then 100 µl of Fc block (biolegend, cat#422302) diluted 1:200 in FACS buffer (PBS containing 0.1% BSA) was added thereto. After leaving for 10 minutes, the cells were stained with anti-CD3, anti-CD4, and anti-CD8 antibodies for 30 minutes in order to measure the contents of T cells. And, the cells were stained with anti-CCR7 and anti-CD45RA antibodies in order to evaluate the degree of cell differentiation; and were stained with anti-CD137 and anti-PD-1 antibodies in order to evaluate their function. Then, flow cytometry was performed with a CytoFLEX flow cytometry device and the results were analyzed with FlowJo version 10.1 software. The antibodies used are listed in Table 2 below.

**Table 2. List of Antibodies**

| Antibody | Supplier | Cat |
|---|---|---|
| CD3-eFluor 450 | Invitrogen | 48-0037-42 |
| CD4-PE | BD | 555347 |
| CD8-APC | BD | 555369 |
| CCR7-APC Cy7 | Biolegend | 353212 |
| CD45RA-BV 421 | Biolegend | 304130 |
| CD137-Alexa Fluor^{®}700 | Biolegend | 309816 |
| PD1-BV 421 | BD | 562516 |

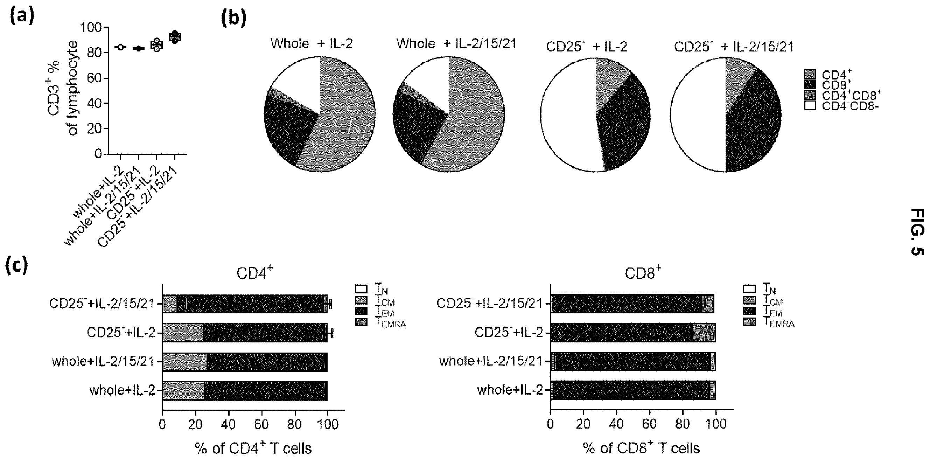

The results of flow cytometry as described above are shown in FIG. 5. In case of the TILs containing CD25⁺ cells, the contents of CD3+ cells in lymphocytes were 84.48% (Whole + IL-2) or 83.39% (Whole + IL-2/15/21). In case of the TILs cultured after removal of CD25⁺ cells, the contents of CD3+ cells in lymphocytes were 89.69% (CD25⁻ + IL-2) or 95.73% (CD25⁻ + IL-2/15/21) and thus the proliferation of CD3+ T-cells therein were further increased (FIG. 5a). And, as a result of evaluating the ratio of CD4+ cells in CD3+ cells, the ratio of CD4+ cells was 57.23% (Whole + IL-2) or 58.22% (Whole + IL-2/15/21) in case of the TILs containing CD25⁺ cells. In case of the TILs cultured after removal of CD25⁺ cells, the ratio of CD4+ cells was decreased to 11.54% (CD25- + IL-2) or 9.31% (CD25- + IL-2/15/21) (FIG. 5b). In addition, the ratio of CD8+ cells in CD3+ cells was 23.27% (Whole + IL-2) or 26.32% (Whole + IL-2/15/21) in case of the TILs containing CD25⁺ cells, whereas in case of the TILs cultured after removal of CD25⁺ cells, the ratio of CD8+ cells in CD3+ cells was 35.46% (CD25⁻ + IL-2) or 41.7% (CD25⁻ + IL-2/15/21) and thus CD25⁻ + IL-2/15/21 shows the highest increase in the ratio of CD8+ cells (FIG. 5b). That is, the TILs cultured in medium containing IL2/15/21 after removal of CD25⁺ cells showed decrease in the CD4 ratio and increase in the CD8 ratio.

The expressions of CD45RA and CCR7 were analyzed by FACS to evaluate the population ratio of T cell Central Memory and T cell Effector Memory, through classifying to Naive (CD45RA+CCR7+), TCM (CD45RA-CCR7+), TEM (CD45RA-CCR7+), and TEMRA (CD45RA+CCR7-). In CD4+ T cells, the TILs cultured in the medium containing IL-2/15/21 after removal of CD25⁺ cells (CD25⁻ + IL-2/15/21) showed decrease in TCM and increase in TEM, compared to the other groups. In CD8+ T cells, the TILs cultured after removal of CD25⁺ cells (CD25⁻ + IL-2 or CD25⁻ + IL-2/15/21) showed the tendency of increase in TEMRA (FIG. 5c).

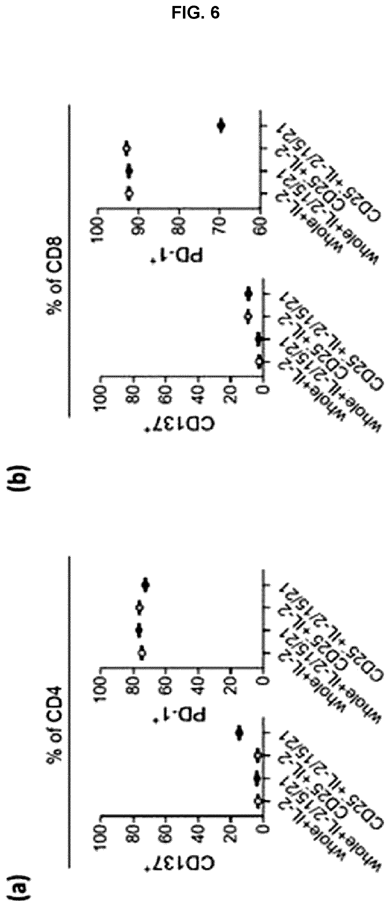

In the expression of CD137 (which is involved in T cell activation as a T cell costimulatory molecule) in CD4+ T cells, the TILs containing CD25⁺ cells showed 3.19% (Whole + IL-2) or 3.78% (Whole + IL-2/15/21) and the TILs cultured after removing CD25⁺ cells showed 3.46% (CD25⁻ + IL-2) or 14.8% (CD25⁻ + IL-2/15/21). In the expression of PD-1 in CD4+ T cells, the TILs containing CD25⁺ cells showed 74.81% (Whole + IL-2) or 76.67% (Whole + IL-2/15/21) and the TILs cultured after removing CD25⁺ cells showed 76.27% (CD25⁻ + IL-2) or 72.70% (CD25⁻ + IL-2/15/21) (FIG. 6a). And, in the expression of CD137 in CD8+ T cells, the TILs containing CD25⁺ cells showed 2.6% (Whole + IL-2) or 2.94% (Whole + IL-2/15/21) and the TILs cultured after removing CD25⁺ cells showed 9.32% (CD25⁻ + IL-2) or 9.1% (CD25⁻ + IL-2/15/21). In the expression of PD-1 in CD8+ T cells, the TILs containing CD25⁺ cells showed 92.33% (Whole + IL-2) or 92.32% (Whole + IL-2/15/21) and the TILs cultured after removing CD25⁺ cells showed 92.92% (CD25⁻ + IL-2) or 69.7% (CD25⁻ + IL-2/15/21) (FIG. 6b). Therefore, it can be seen that, when the TILs cultured after removing CD25⁺ cells, especially when the TILs cultured in a medium containing the three cytokines IL2/15/21 after removing CD25⁺ cells, the expression of CD137, an activation marker, increases and the expression of PD-1 decreases. In addition, it can be confirmed that there is no significant difference between the use of a medium containing IL-2/15/21 and the use of a medium containing IL-2 alone, in culturing the TILs containing CD25⁺ cells.

### Test Example 5: Evaluation on tumor cell-killing ability of TILs

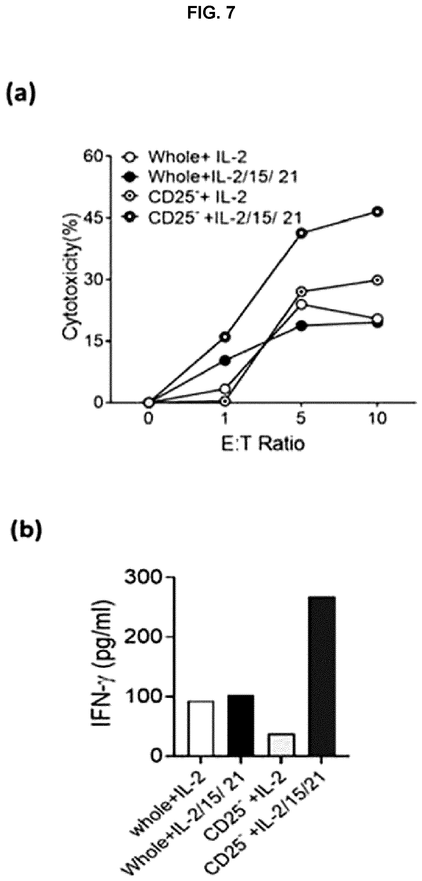

After cancer cells and the TILs (CD25- + IL2, CD25⁻ + IL2/15/21, Whole + IL2, and Whole + IL2/15/21) were cultured at 37°C and 5% CO₂ conditions for 4 hours, the tumor cell-killing abilities thereof were evaluated in the same manner as in Test Example 2. The results are as shown in FIG. 7. As can be seen from the results of FIG. 7, when the ratio of cancer cells:TILs is 1:10, the TILs containing CD25⁺ cells showed the cytotoxicity of 20.49% (Whole + IL-2) or 19.58% (Whole + IL-2/ 15/21) and the TILs cultured after removing CD25⁺ cells showed 29.88% of cytotoxicity (CD25⁻ + IL-2) or 46.5% of cytotoxicity (CD25⁻ + IL-2/15/21) (FIG. 7a). That is, the TILs cultured after removing CD25⁺ cells, especially the TILs cultured in a medium containing the three cytokines IL2/15/21 after removing CD25⁺ cells, showed the highest tumor cell-killing ability. In the above evaluation on tumor cell-killing ability, the cell culture medium cultured for 4 hours was collected and ELISA (enzyme-linked immunosorbent assay) analysis thereon was performed using a Human IFN-γ ELISA Kit (BD, Cat# 555142). As a result, the TILs containing CD25⁺ cells secreted 93.3 pg/ml of IFN-γ (Whole + IL-2) or 103.41 pg/ml of IFN-γ (Whole + IL-2/15/21) and the TILs cultured after removing CD25⁺ cells secreted 38.45 pg/ml of IFN-γ (CD25⁻ + IL-2) or 268.11 pg/ml of IFN-γ (CD25⁻ + IL-2/15/21) (FIG. 7b).

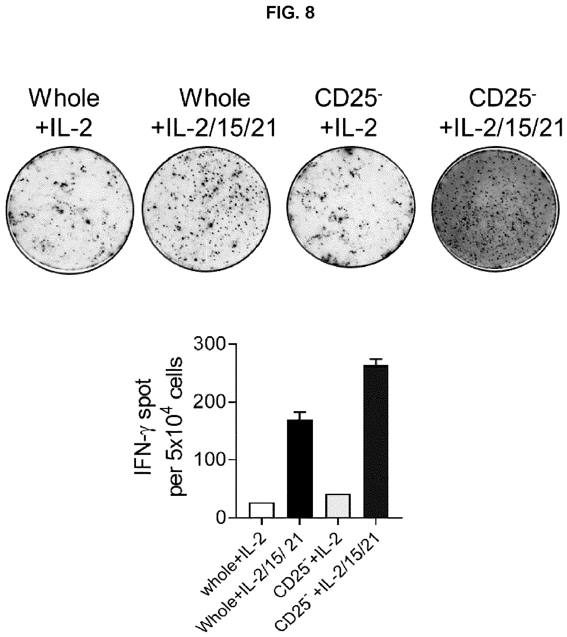

In addition, in order to confirm the IFN-γ secreted by the TILs per se, ELISPOT (Enzyme-Linked ImmunoSpot) analysis was performed on the TILs without cancer cells, using a Human IFN-γELISpotPRO kit (mabtech, 3420-4HST-2). As a result, the TILs containing CD25⁺ cells secreted 27 of IFN-γ (Whole + IL-2) or 160 of IFN-γ (Whole + IL-2/15/21) and the TILs cultured after removing CD25⁺ cells secreted 42 of IFN-γ (CD25- + IL-2) or 271 of IFN-γ (CD25- + IL-2/15/21) (FIG. 8). Therefore, the TILs cultured after removing CD25⁺ cells, especially the TILs cultured in a medium containing the three cytokines IL2/15/21 after removing CD25⁺ cells, showed the highest IFN-γ secretion.

### Example 3: Preparation of tumor-infiltrating lymphocytes (TILs)

Tumor tissues were obtained from ovarian cancer patients. Written informed consent was obtained from the patients before participating in this study. This study was approved by the institutional review board of CHA Bundang Medical Center.

### (1) Isolation of single cells from tumor tissues

The tumor tissues isolated from the body were sectioned into 1-3 mm³ fragments and then single cells were isolated from the sectioned tumor tissues using a gentleMACS Dissociator. The isolated single cells were maintained in the culture medium obtained by mixing the RPMI 1640 GlutaMAX medium shown in Table 1 above (Thermo Scientific, 72400-047) and the AIM-V serum-free medium (Gibco, 12055-091) in a 1:1 ratio.

### (2) Cell culture (pre-REP)

The single cells obtained in (1) above were cultured for 14 days in the culture medium supplemented with 20 µg/ml of an anti-GITR antibody (BPS bioscience, 79053), 10 µg/ml of an anti-CD3/28 antibody (stem cell, 10990), 3,000 U/ml of IL-2 (PEPROTECH, 200-02), and 10 µg/ml of a PD-1 inhibitor (J116 monoclonal antibody, BioXcell).

The anti-GITR antibody, IL-2, and the PD-1 inhibitor were added to the culture medium on Day 0 of culture. Every 5 days after the start of culture, the culturing was carried out by replacing approximately 1/2 of the culture medium with a medium supplemented with the anti-CD3/28 antibody and IL-2. In addition, the culturing was carried out while adding the culture medium supplemented with IL-2 every 2 days between the medium replacements every 5 days. Specifically, on Day 0 of culture, the single cells obtained in (1) above (1×10⁶ cells/2ml) were added to a G-rex 24-well plate (wilsonwolf, 80192M) and then cultured in 2 ml of the culture medium supplemented with 20 µg/ml of the anti-GITR antibody (BPS bioscience, 79053), 3,000 U/ml of IL-2 (PEPROTECH, 200-02) and 10 µg/ml of the PD-1 inhibitor (J116 monoclonal antibody, BioXcell). After 5 days (on the 5th day of pre-REP culture), 1 ml of the culture medium (i.e., the half thereof) was removed. 1 ml of the culture medium supplemented with 10 µg/ml of the anti-CD3/28 antibody (stem cell, 10990) and 3,000 U/ml of IL-2 was added thereto (i.e., 2 ml in total) and then cultured. After 2 days (on the 7th day of pre-REP culture), 1 ml of the culture medium (i.e., the half thereof) was removed. 1 ml of the culture medium supplemented with 3,000 U/ml of IL-2 was added (total 2 ml) and cultured. After 3 days (on the 10th day of pre-REP culture), 1 ml of the culture medium (i.e., the half thereof) was removed. 1 ml of the culture medium supplemented with 10 µg/ml of the anti-CD3/28 antibody (stem cell, 10990) and 3,000 U/ml of IL-2 was added thereto (i.e., 2 ml in total) and then cultured. After 2 days (on the 12th day of pre-REP culture), 1 ml of the culture medium (i.e., the half thereof) was removed. 1 ml of the culture medium supplemented with 3,000 U/ml of IL-2 was added thereto (i.e., 2 ml in total) and then cultured for 2 days. That is, the culturing was carried out for 14 days in total (i.e., pre-REP culture for 14 days in total).

### (3) Rapid Expansion Protocol (REP)

5 ml of the culture medium supplemented with 2 µg/ml of phytohemagglutinin (PHA) and 3,000 U/ml of IL-2 (PEPROTECH, 200-02) was added to a T25 Flask and the TILs obtained in (2) (2×10⁵ cells) and peripheral mononuclear cells (irradiated with 55Gy, 2×10⁷ cells) were co-cultured therein. After 5 days (on the 5th day of REP culture), 5 ml of the culture medium supplemented with 3,000 U/ml of IL-2 was added thereto (i.e., 10 ml in total) and then cultured. After 2 days (on the 7th day of REP culture), 20 ml of the culture medium supplemented with 3,000 U/ml of IL-2 was added thereto (i.e., 30 ml in total) and then cultured. After 3 days (on the 10th day of REP culture), the cells were collected in a 50ml conical tube and centrifuged at 1,200 rpm for 3 minutes. After removing 15 ml of the culture medium (i.e., the half thereof), 15 ml of the culture medium supplemented with 3,000 U/ml of IL-2 was added thereto (i.e., 30 ml in total) and then cultured. After 2 days (on the 12th day of REP culture), 60 ml of the culture medium supplemented with 3,000 U/ml of IL-2 was added thereto (i.e., 90 ml in total) and then cultured for 2 days. That is, the culturing was carried out for 14 days in total (i.e., REP culture for 14 days in total).

### Example 4: Preparation of tumor-infiltrating lymphocytes (TILs)

### (1) Cell culture (pre-REP)

The TILs were prepared by culturing according to the same manner as in (2) of Example 3 for 30 days in total, in the culture medium supplemented with 10 µg/ml of an anti-CD3/28 antibody (stem cell, 10990), 3,000 U/ml of IL-2 (PEPROTECH, 200-02), 180 U/ml of IL-15 (PEPROTECH, 200-02), 40 U/ml of IL-21 (PEPROTECH, 200-21), and 10 µg/ml of a PD-1 inhibitor (J116 monoclonal antibody, BioXcell).

IL-2/IL-15/IL-21 and the PD-1 inhibitor were added to the culture medium on Day 0 of culture. Every 5 days after the start of culture, the culturing was carried out by replacing approximately 1/2 of the culture medium with a medium supplemented with the anti-CD3/28 antibody and IL-2/IL-15/IL-21. In addition, the culturing was carried out while adding the culture medium supplemented with IL-2/IL-15/IL-21 every 2 days between the medium replacements every 5 days. Specifically, on Day 0 of culture, the cells obtained in the above (1×10⁶ cells/2ml) were added to a G-rex 24-well plate (wilsonwolf, 80192M) and then cultured in 2 ml of the culture medium supplemented with 3,000 U/ml of IL-2 (PEPROTECH, 200-02), 180 U/ml of IL-15 (PEPROTECH, 200-02), 40 U/ml of IL-21 (PEPROTECH, 200-21), and 10 µg/ml of the PD-1 inhibitor (J116 monoclonal antibody, BioXcell). After 5 days (on the 5th day of pre-REP culture), 1 ml of the culture medium (i.e., the half thereof) was removed. 1 ml of the culture medium supplemented with 10 µg/ml of the anti-CD3/28 antibody (stem cell, 10990), 3,000 U/ml of IL-2 (PEPROTECH, 200-02), 180 U/ml of IL-15 (PEPROTECH, 200-02), and 40 U/ml of IL-21 (PEPROTECH, 200-21) was added thereto (i.e., 2 ml in total) and then cultured. After 2 days (on the 7th day of pre-REP culture), 1 ml of the culture medium (i.e., the half thereof) was removed. 1 ml of the culture medium supplemented with 3,000 U/ml of IL-2, 180 U/ml of IL-15 (PEPROTECH, 200-02), and 40 U/ml of IL-21 (PEPROTECH, 200-21) was added (total 2 ml) and cultured. After 3 days (on the 10th day of pre-REP culture), 1 ml of the culture medium (i.e., the half thereof) was removed. 1 ml of the culture medium supplemented with 10 µg/ml of the anti-CD3/28 antibody (stem cell, 10990), 3,000 U/ml of IL-2 (PEPROTECH, 200-02), 180 U/ml of IL-15 (PEPROTECH, 200-02), and 40 U/ml of IL-21 (PEPROTECH, 200-21) was added thereto (i.e., 2 ml in total) and then cultured. After 2 days (on the 12th day of pre-REP culture), 1 ml of the culture medium (i.e., the half thereof) was removed. 1 ml of the culture medium supplemented with 3,000 U/ml of IL-2, 180 U/ml of IL-15 (PEPROTECH, 200-02), and 40 U/ml of IL-21 (PEPROTECH, 200-21) was added thereto (i.e., 2 ml in total) and then cultured for 2 days. That is, the culturing was carried out for 14 days in total (i.e., pre-REP culture for 14 days in total). The pre-REP culture after 14 days was performed in the same manner as the pre-REP culture for 14 days. The TILs obtained by the pre-REP culture for 14 days as described above are referred to as "Anti-PD-1/GITR+IL-2/15/21" in the test examples below.

### (2) Rapid Expansion Protocol (REP)

REP culture for the Anti-PD-1/GITR+IL-2/15/21 was carried out by culturing according to the same manner as in (2) of Example 2 for 14 days in total, except for using 3,000 U/ml of IL-2 (PEPROTECH, 200-02), 180 U/ml of IL-15 (PEPROTECH, 200-02), and 40 U/ml of IL-21 (PEPROTECH, 200-21) as cytokines.

### Comparative Example 2: Preparation of tumor-infiltrating lymphocytes (TILs)

### (1) Preparation of Control TILs

TILs were prepared without adding an anti-GITR-antibody and a PD-1 inhibitor to the medium, in (2) of Example 3. That is, a 14-day pre-REP culture was carried out according to the same manner as in (2) of Example 3, except for adding the single cells isolated from tumor tissue (1×10⁶ cells/2ml) to a G-rex 24-well plate (wilsonwolf, 80192M) on Day 0 of culture and using 3,000 U/ml of IL-2 (PEPROTECH, 200-02) alone. The obtained TILs are referred to as "Control TILs" in the test examples below.

REP culture for the Control TILs was carried out by culturing according to the same manner as in (3) of Example 3 for 14 days in total, except for using 3,000 U/ml of IL-2 (PEPROTECH, 200-02) as a cytokine.

### (2) Preparation of Anti-PD-1 TILs

TILs were prepared without adding an anti-GITR-antibody to the medium, in (2) of Example 3. That is, a 14-day pre-REP culture was carried out according to the same manner as in (2) of Example 3, except for adding the single cells isolated from tumor tissue (1×10⁶ cells/2ml) to a G-rex 24-well plate (wilsonwolf, 80192M) on Day 0 of culture and using 3,000 U/ml of IL-2 (PEPROTECH, 200-02) and 10 µg/ml of the PD-1 inhibitor (J116 monoclonal antibody, BioXcell). The obtained TILs are referred to as "Anti-PD-1 TILs" in the test examples below.

REP culture for the Anti-PD-1 TILs was carried out by culturing according to the same manner as in (3) of Example 3 for 14 days in total, except for using 3,000 U/ml of IL-2 (PEPROTECH, 200-02) as a cytokine.

### (3) Preparation of Anti-PD-1+IL-2/15/21 TILs

TILs were prepared without adding an anti-GITR-antibody to the medium, in (2) of Example 3, using 3,000 U/ml of IL-2 (PEPROTECH, 200-02), 180 U/ml of IL-15 (PEPROTECH, 200-02), and 40 U/ml of IL-21 (PEPROTECH, 200-21) as cytokines. That is, a 14-day pre-REP culture was carried out according to the same manner as in (2) of Example 3, except for adding the single cells isolated from tumor tissue (1×10⁶ cells/2ml) to a G-rex 24-well plate (wilsonwolf, 80192M) on Day 0 of culture and using 10 µg/ml of the PD-1 inhibitor (J116 monoclonal antibody, BioXcell), 3,000 U/ml of IL-2 (PEPROTECH, 200-02), 180 U/ml of IL-15 (PEPROTECH, 200-02), and 40 U/ml of IL-21 (PEPROTECH, 200-21). The obtained TILs are referred to as "Anti-PD-1+IL-2/15/21 TILs" in the test examples below.

REP culture for the Anti-PD-1+IL-2/15/21 TILs was carried out by culturing according to the same manner as in (2) of Example 4 for 14 days in total, except for using 3,000 U/ml of IL-2 (PEPROTECH, 200-02), 180 U/ml of IL-15 (PEPROTECH, 200-02), and 40 U/ml of IL-21 (PEPROTECH, 200-21) as cytokines.

Test Example 6: Evaluation on tumor cell-killing ability of the TILs obtained through REP culture

The tumor cell-killing abilities of the TILs obtained by the REP culture for 14 days in Example 1; and the TILs obtained by the REP culture for 14 days in Example 3 were evaluated. Specifically, cancer cells from the tumor tissue of an ovarian cancer patient were diluted in PBS to 2×10⁵ cells/ml and then fluorescently stained with 5 µM of Far-red (Invitrogen, C34564). After separating the stained cells by centrifugation at 2000 rpm for about 3 minutes, the stained cancer cells were added in the concentration of 2×10⁵ cells/ml to the cancer cell culture medium [obtained by mixing the RPMI 1640 GlutaMAX medium (Thermo Scientific, 72400-047) supplemented with the excipients of Table 1 above and the AIM-V serum-free medium (Gibco, 12055-091) in a 1:1 ratio] and then 100 µl thereof was added into each well of a v-bottom 96-well plate. The TILs obtained by the REP culture for 14 days in Example 1 and the TILs obtained by the REP culture for 14 days in Example 3 were diluted in the cancer cell culture medium to 2×10⁶ cells/ml, respectively, and then added into each well at a cancer cell:TILs ratio of 1:1, 1:5, and 1:10. The amount of culture medium containing cells per well was set to 200 µl. After culturing at 37°C and 5% CO₂ conditions for 24 hours, living cancer cells were observed through a fluorescence microscope. The cell mixture was pelleted to remove the culture medium and then stained for 15 minutes at room temperature with 7-AAD (7-amino-actinomycin D, Thermo, A1310) for staining dead cells. The stained mixture was washed once with phosphate-buffered saline (PBS) and then suspended in a FACS buffer. The percentage (%) of 7AAD+ cells in Far-red+ cells was measured using a CytoFLEX flow cytometry (Beckman Coulter) and analyzed with a FlowJo version 10.1 software (Treestar inc, Ashland). Analysis of the results taken with the fluorescence microscope was carried out using the image J program and the percentage of living cells was analyzed by assuming that the percentage of the group containing only cancer cells (without co-culture with TILs) was set to 100%.

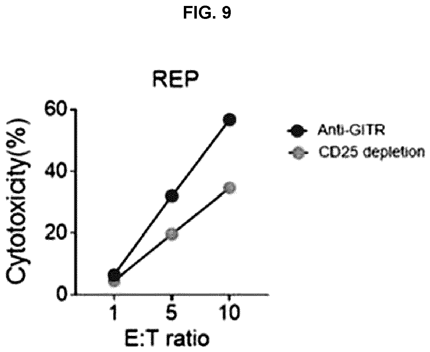

The results obtained by evaluating the tumor cell-killing ability as described above are shown in FIG. 9. As can be seen from the results in FIG. 9, when the ratio of cancer cells:TILs is 1:10, the TILs obtained by the REP culture for 14 days in Example 1 (i.e., CD25-depleted TILs) showed the cytotoxicity of 34.6% and the TILs obtained by the REP culture for 14 days in Example 3 (i.e., anti-GITR TILs) showed the cytotoxicity of 56.7%.

### Test Example 7: Evaluation on proliferation of TILs

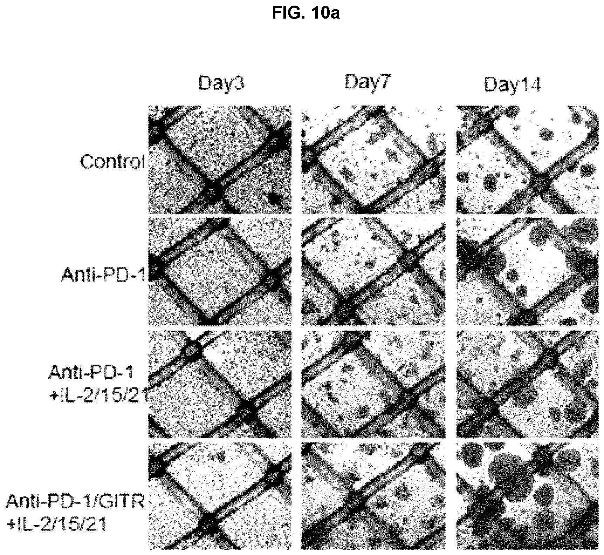

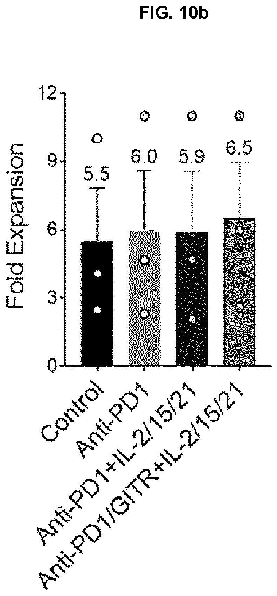

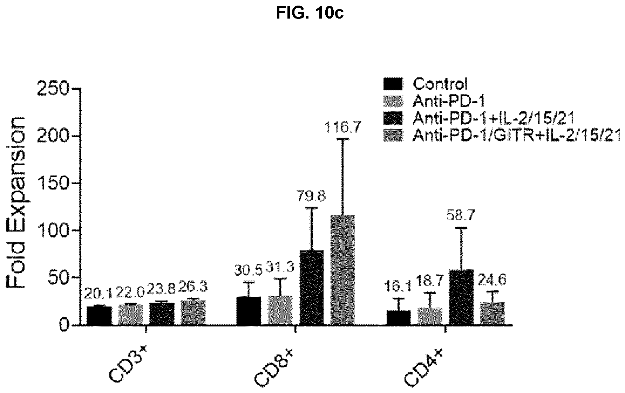

While Control TILs, Anti-PD-1 TILs, Anti-PD-1+IL-2/15/21 TILs, and Anti-PD-1/GITR+IL-2/15/21 TILs were grown for 14 days, the number of proliferated cells was measured. The number of cells was measured with a LUNA cell counter after staining with Acridine Orange/Propidium Iodide staining reagent (logos bio, F23001). The results are as shown in FIG. 10. As can be seen from the results of FIG 10, the Anti-PD-1/GITR+IL-2/15/21 TILs were best proliferated, showing the clumping, on the 14th day of culture (FIG. 10a). When grown for 14 days, the Control TILs showed 5.5 times of proliferation, the Anti-PD-1 TILs showed 6.0 times of proliferation, the Anti-PD-1+IL-2/15/21 TILs showed 5.9 times of proliferation, and the Anti-PD-1/GITR+IL-2/15/21 TILs showed 6.5 times of proliferation (FIG. 10(b)). And, when grown for 14 days, the Control TILs showed 30.5 times of CD8⁺ cell proliferation, the Anti-PD-1 TILs showed 31.3 times of CD8⁺ cell proliferation, the Anti-PD-1+IL-2/15/21 TILs showed 79.8 times of CD8⁺ cell proliferation, and the Anti-PD-1/GITR+IL-2/15/21 TILs showed 116.7 times of CD8⁺ cell proliferation (FIG. 10(c)). From the above results, it can be confirmed that, when cultured in combination of an anti-GITR antibody with cytokines, the TILs exhibited excellent proliferation ability.

### Test Example 8: Evaluation on differentiation and activity of the immune cells

The cell culture of Control TILs, the cell culture of Anti-PD-1 TILs, the cell culture of Anti-PD-1+IL-2/15/21 TILs, and the cell culture of Anti-PD-1/GITR+IL-2/15/21 TILs were respectively centrifuged at 1,500 rpm for 5 minutes at 4°C to remove each supernatant and then 100 µl of Fc block (biolegend, cat#422302) diluted 1:200 in FACS buffer (PBS containing 0.1% BSA) was added thereto. After leaving for 10 minutes, the cells were stained with anti-CD3, anti-CD4, and anti-CD8 antibodies for 30 minutes in order to measure the contents of T cells. And, the cells were stained with anti-CD137 and anti-Granzyme B antibodies in order to evaluate their function; and were stained with an anti-CD4 antibody for 30 minutes, and then with an anti-Foxp3 antibody, in order to evaluate the contents of Treg cells. Then, flow cytometry was performed with a CytoFLEX flow cytometry device and the results were analyzed with FlowJo version 10.1 software. The antibodies used are listed in Table 3 below.

**Table 3. List of Antibodies**

| Antibody | Supplier | Cat |
|---|---|---|
| CD3-eFluor 450 | Invitrogen | 48-0037-42 |
| CD4-PE | BD | 555347 |
| CD8-APC | BD | 555369 |
| CD137-Alexa Fluor 700 | Biolegend | 309816 |
| Granzyme B | BD | 562516 |
| CCR7-BV605 | Biolegend | 353224 |
| CD45RA-BV510 | Biolegend | 304142 |
| PD1-Alexa Fluor 700 | Biolegend | 329952 |
| FOXP3-Alexa Fluor 700 | Invitrogen | 56-4776-41 |

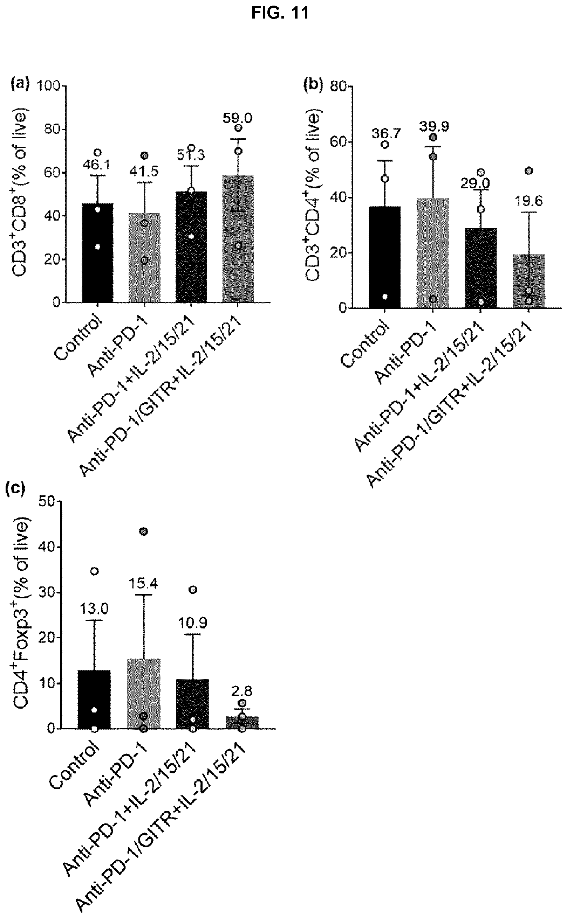

The results of flow cytometry as described above are shown in FIG. 11. The contents of CD3⁺CD8⁺ cells in the living cells were 46.1% (Control TILs), 41.5% (Anti-PD-1 TILs), 51.3% (Anti-PD-1+IL-2/15/21 TILs), and 59% (Anti-PD-1/GITR+IL-2/15/21 TILs), respectively, and thus the proliferation of CD3⁺CD8⁺ T-cells therein were further increased after the anti-GITR-antibody treatment (FIG. 11a). And, as a result of evaluating the ratio of CD3⁺CD4⁺ cells in the living cells, the Control TILs showed 36.7%, the Anti-PD-1 TILs showed 39.9%, the Anti-PD-1+IL-2/15/21 TILs showed 29%, and the Anti-PD-1/GITR+IL-2/15/21 TILs showed 19.6%, and thus the CD3⁺CD4⁺ cells therein were decreased after the anti-GITR-antibody treatment (FIG. 11b). The ratios of CD4⁺Foxp3⁺ Treg cells in the living cells were 13.0% (Control TILs), 15.4% (Anti-PD-1 TILs), 10.9% (Anti-PD-1+IL-2/15/21 TILs), and 2.6% (Anti-PD-1/GITR+IL-2/15/21 TILs), respectively, and thus the CD4⁺Foxp3⁺ Treg cells therein were decreased after the anti-GITR-antibody treatment (FIG. 11c). That is, the TILs obtained by treatment with an anti-GITR-antibody showed decrease in the ratio of CD4⁺ and Treg cells and increase in the ratio of CD8⁺ cells.

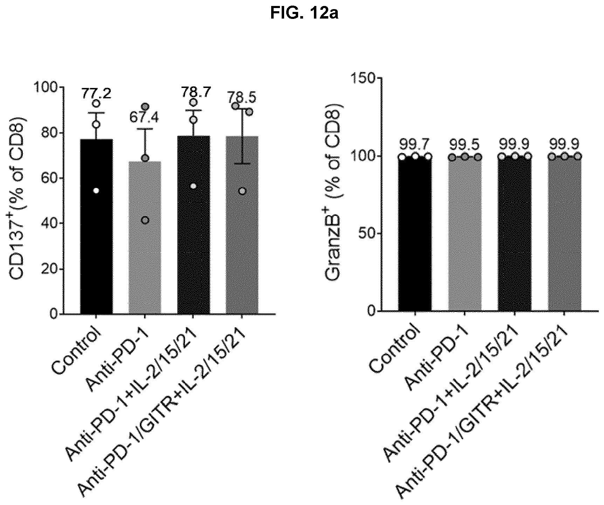

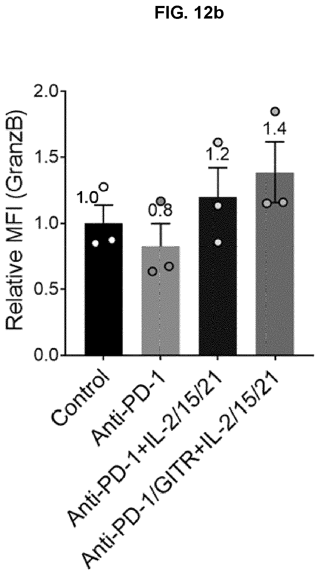

In the expression of CD137 (which is involved in T cell activation as a T cell costimulatory molecule) in CD8⁺ T cells, the Control TILs showed 77.2%, the Anti-PD-1 TILs showed 67.4%, the Anti-PD-1+IL-2/15/21 TILs showed 78.7%, and the Anti-PD-1/GITR+IL-2/15/21 TILs showed 78.5%. In the expression of Granzyme B therein, the Control TILs showed 99.7%, the Anti-PD-1 TILs showed 99.5%, the Anti-PD-1+IL-2/15/21 TILs showed 99.9%, and the Anti-PD-1/GITR+IL-2/15/21 TILs showed 99.9% (FIG. 12a). Compared to the Control TILs, the expression level of Granzyme B is increased by 0.8 times in the Anti-PD-1 TILs, 1.2 times in Anti-PD-1+IL-2/15/21 TILs, and 1.4 times in the Anti-PD-1/GITR+IL-2/15/21 TILs (FIG. 12b). Therefore, it can be seen that, when the TILs obtained by treatment with an anti-GITR-antibody were cultured in a medium containing IL2/15/21, the expression of Granzyme B, an activation marker, increases.

### Test Example 9: Evaluation on tumor cell-killing ability of TILs

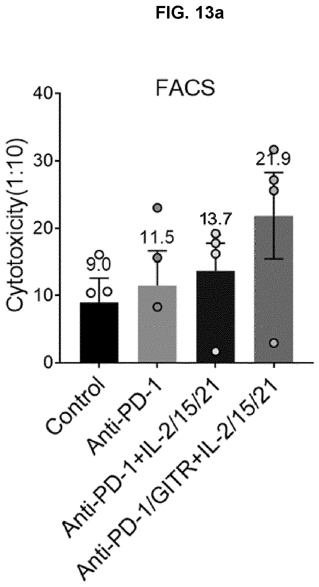

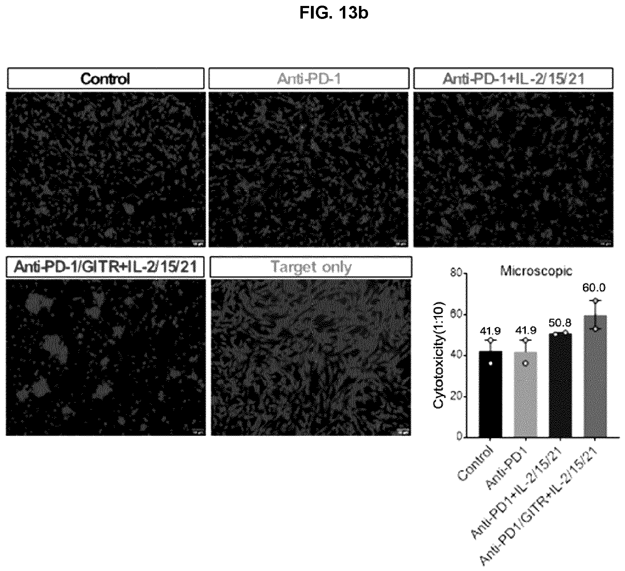

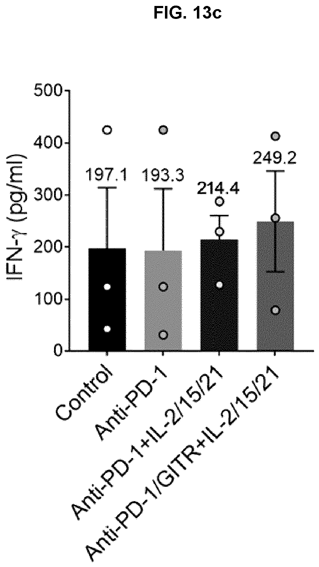

After cancer cells and the TILs (Control TILs, Anti-PD-1 TILs, Anti-PD-1+IL-2/15/21 TILs, and Anti-PD-1/GITR+IL-2/15/21 TILs) were cultured at 37°C and 5% CO₂ conditions for 24 hours, the tumor cell-killing abilities thereof were evaluated in the same manner as in Test Example 6. The results are as shown in FIG. 13. As can be seen from the results of FIG. 13, when the ratio of cancer cells:TILs is 1:10, the Control TILs showed 9.0% of cytotoxicity, the Anti-PD-1 TILs showed 11.5% of cytotoxicity, the Anti-PD-1+IL-2/15/21 TILs showed 13.7% of cytotoxicity, and the Anti-PD-1/GITR+IL-2/15/21 TILs showed 21.7% of cytotoxicity, when measured using FACs (FIG. 13a). When analyzed through microscopic observation, the Control TILs showed 41.9% of cytotoxicity, the Anti-PD-1 TILs showed 41.9% of cytotoxicity, the Anti-PD-1+IL-2/15/21 TILs showed 50.8% of cytotoxicity, and the Anti-PD-1/GITR+IL-2/15/21 TILs showed 60% of cytotoxicity (FIG. 13b). That is, the TILs obtained by treatment with an anti-GITR-antibody showed the highest tumor cell-killing ability. In the above evaluation on tumor cell-killing ability, the cell culture medium cultured for 24 hours was collected and ELISA (enzyme-linked immunosorbent assay) analysis thereon was performed using a Human IFN-γ ELISA Kit (BD, Cat# 555142). As a result, the Control TILs secreted 197.1 pg/ml of IFN-γ, the Anti-PD-1 TILs secreted 193.3 pg/ml of IFN-γ, the Anti-PD-1+IL-2/15/21 TILs secreted 214.4 pg/ml of IFN-γ, and the Anti-PD-1/GITR+IL-2/15/21 TILs secreted 249.2 pg/ml of IFN-γ (FIG. 13c). Therefore, the TILs obtained by treatment with an anti-GITR-antibody showed the highest IFN-γ secretion.

### Test Example 10: Evaluation on proliferation of TILs

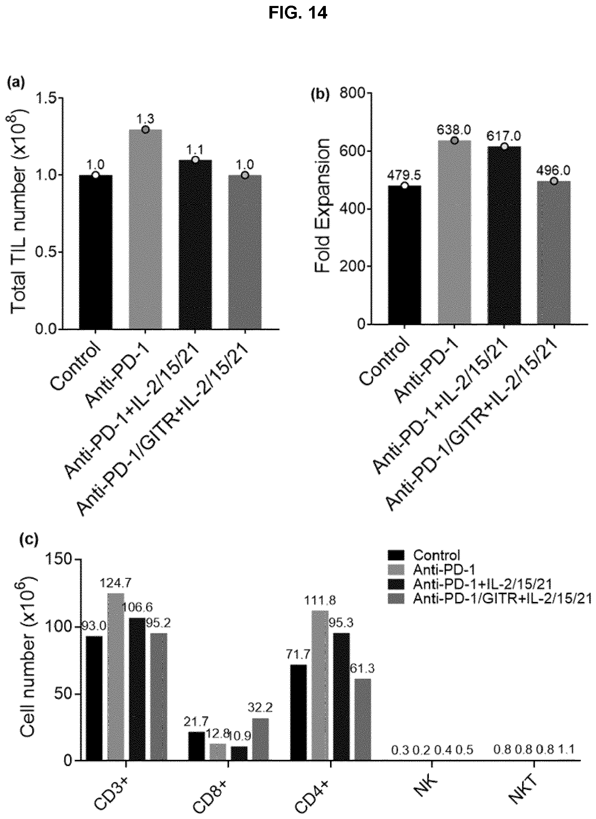

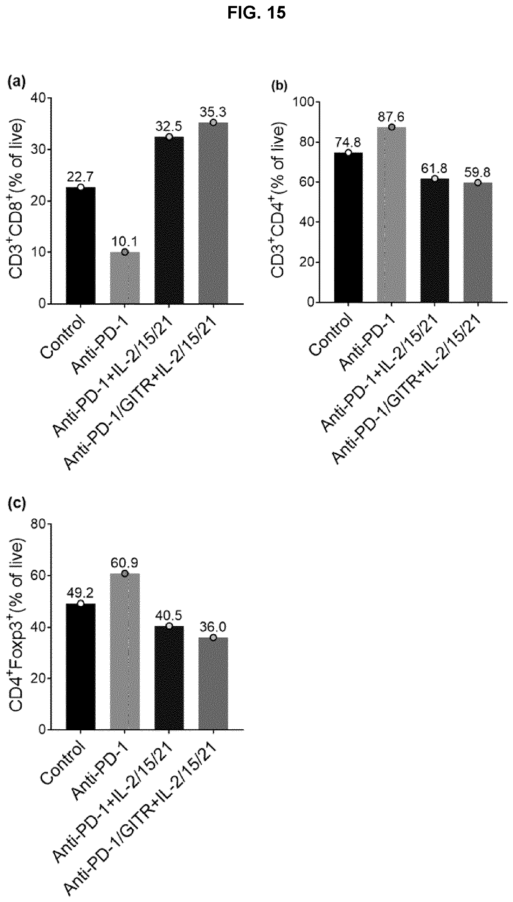

While the Control TILs, the Anti-PD-1 TILs, the Anti-PD-1+IL-2/15/21 TILs, and the Anti-PD-1/GITR+IL-2/15/21 TILs were subject to the REP culture for 14 days as in the above, the number of proliferated cells was measured. The number of cells was measured with a LUNA cell counter after staining with Acridine Orange/Propidium Iodide staining reagent (logos bio, F23001). The results are as shown in FIG. 14. As can be seen from the results of FIG 14, the Control TILs were proliferated from 2X10⁵ cells on Day 0 of culture to 9.59×10⁷ cells (479.5 times) on the 14th day of culture. The Anti-PD-1 TILs were proliferated from 2×10⁵ cells on Day 0 of culture to 1.27×10⁸ cells (638 times) on the 14th day of culture. The Anti-PD-1+IL-2/15/21 TILs were proliferated from 2X10⁵ cells on Day 0 of culture to 1.1×10⁸ cells (617 times) on the 14th day of culture. The Anti-PD-1/GITR+IL-2/15/21 TILs were proliferated from 2X10⁵ cells on Day 0 of culture to 9.99×10⁷ cells (496 times) on the 14th day of culture (FIG. 14a, 14b). In the CD8⁺ cells after the REP culture for 14 days, the Control TILs showed 21.7 times of proliferation, the Anti-PD-1 TILs showed 12.8 times of proliferation, the Anti-PD-1+IL-2/15/21 TILs showed 10.9 times of proliferation, and the Anti-PD-1/GITR+IL-2/15/21 TILs showed 32.2 times of proliferation (FIG. 15c). From the above results, it can be seen that the TILs obtained by treatment with an anti-GITR antibody showed 1.5 times of increase in the number of CD8⁺ cells, compared to the Control TILs.

Test Example 11: Evaluation on differentiation and activity of the immune cells in the TILs obtained by the REP culture for 14 days

For the Control TILs, the Anti-PD-1 TILs, the Anti-PD-1+IL-2/15/21 TILs, and the Anti-PD-1/GITR+IL-2/15/21 TILs which were obtained by the REP culture for 14 days, flow cytometry was carried out in the same manner as Test Example 8, and the results thereof are shown in FIG. 15. The contents of CD3⁺CD8⁺ cells in the living cells were 22.7% (Control TILs), 10.1% (Anti-PD-1 TILs), 32.5% (Anti-PD-1+IL-2/15/21 TILs), and 35.3% (Anti-PD-1/GITR+IL-2/15/21 TILs), respectively, and thus the proliferation of CD3⁺CD8⁺ T-cells therein were further increased after the anti-GITR-antibody treatment (FIG. 15a). And, as a result of evaluating the ratio of CD3⁺CD4⁺ cells in the living cells, the Control TILs showed 74.8%, the Anti-PD-1 TILs showed 87.6%, the Anti-PD-1+IL-2/15/21 TILs showed 61.8%, and the Anti-PD-1/GITR+IL-2/15/21 TILs showed 59.8%, and thus the CD3⁺CD4⁺ cells therein were decreased after the anti-GITR-antibody treatment (FIG. 15b). The ratios of CD4⁺Foxp3⁺ Treg cells in the living cells were 49.2% (Control TILs), 60.9% (Anti-PD-1 TILs), 40.5% (Anti-PD-1+IL-2/15/21 TILs), and 46% (Anti-PD-1/GITR+IL-2/15/21 TILs), respectively, and thus the CD4⁺Foxp3⁺ Treg cells therein were decreased after the anti-GITR-antibody treatment (FIG. 15c). That is, even after the REP culture for 14 days, the TILs obtained by treatment with an anti-GITR-antibody showed decrease in the ratio of CD4⁺ and Treg cells and increase in the ratio of CD8⁺ cells.

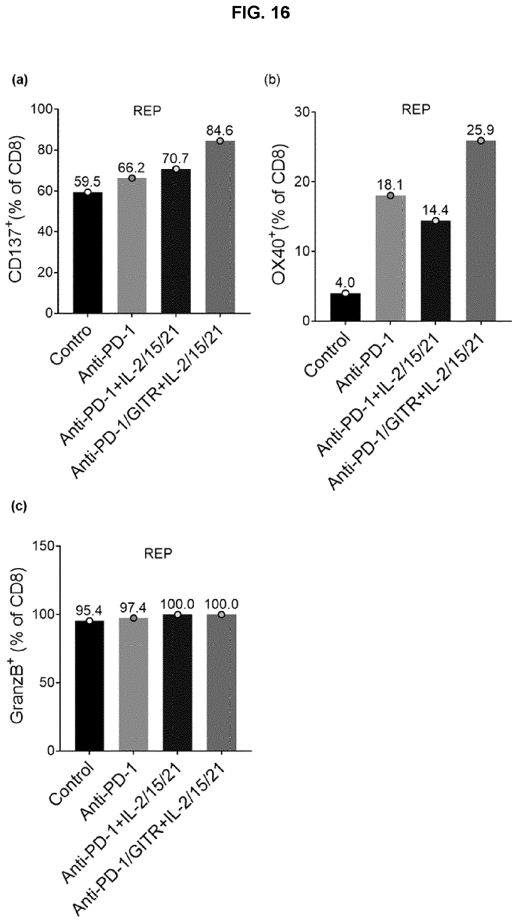

In the expression of CD137 (which is involved in T cell activation as a T cell costimulatory molecule) in CD8⁺ T cells, the Control TILs showed 59.5%, the Anti-PD-1 TILs showed 66.2%, the Anti-PD-1+IL-2/15/21 TILs showed 70.7%, and the Anti-PD-1/GITR+IL-2/15/21 TILs showed 84.6%. In the expression of OX-40 therein, the Control TILs showed 4%, the Anti-PD-1 TILs showed 18.1%, the Anti-PD-1+IL-2/15/21 TILs showed 14.4%, and the Anti-PD-1/GITR+IL-2/15/21 TILs showed 25%. In the expression of Granzyme B therein, the Control TILs showed 95.4%, the Anti-PD-1 TILs showed 97.4%, the Anti-PD-1+IL-2/15/21 TILs showed 100%, and the Anti-PD-1/GITR+IL-2/15/21 TILs showed 100% (FIG. 16). Therefore, it can be seen that, even after the REP culture for 14 days, the TILs obtained by treatment with an anti-GITR-antibody showed increase in the expression of CD137 and OX-40, an activation marker.

Test Example 12: Evaluation on tumor cell-killing ability of the TILs obtained by the REP culture for 14 days

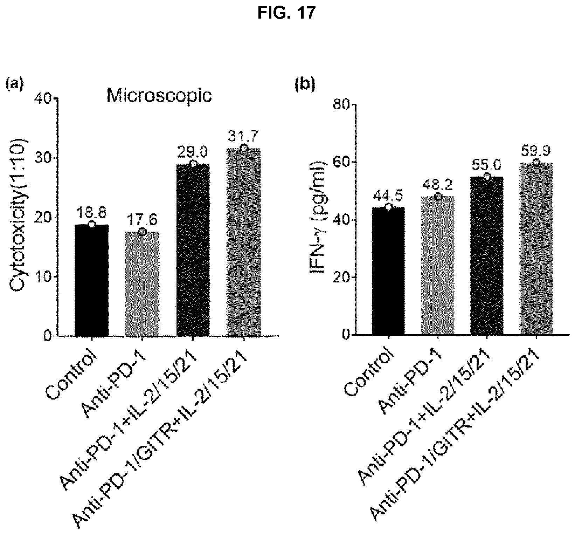

After cancer cells and the TILs (Control TILs, Anti-PD-1 TILs, Anti-PD-1+IL-2/15/21 TILs, and Anti-PD-1/GITR+IL-2/15/21 TILs, which were obtained by the REP culture for 14 days) were cultured at 37°C and 5% CO₂ conditions for 24 hours, the tumor cell-killing abilities thereof were evaluated in the same manner as in Test Example 6. The results are as shown in FIG. 17. As can be seen from the results of FIG. 17, when the ratio of cancer cells:TILs is 1:10, the Control TILs showed 18.8% of cytotoxicity, the Anti-PD-1 TILs showed 17.6% of cytotoxicity, Anti-PD-1+IL-2/15/21 TILs showed 29.0% of cytotoxicity, and the Anti-PD-1/GITR+IL-2/15/21 TILs showed 31.7% of cytotoxicity (FIG. 17a). That is, even after the REP culture for 14 days, the TILs obtained by treatment with an anti-GITR-antibody showed the highest tumor cell-killing ability. In the above evaluation on tumor cell-killing ability, the cell culture medium cultured for 24 hours was collected and ELISA (enzyme-linked immunosorbent assay) analysis thereon was performed using a Human IFN-γ ELISA Kit (BD, Cat# 555142). As a result, the Control TILs secreted 44.5 pg/ml of IFN-γ, the Anti-PD-1 TILs secreted 48.2 pg/ml of IFN-γ, the Anti-PD-1+IL-2/15/21 TILs secreted 55.0 pg/ml of IFN-γ, and the Anti-PD-1/GITR+IL-2/15/21 TILs secreted 59.9 pg/ml of IFN-γ (FIG. 17b). Therefore, even after the REP culture for 14 days, the TILs obtained by treatment with an anti-GITR-antibody showed the highest IFN-γ secretion.

## Claims

1. A method for preparing tumor-infiltrating lymphocytes, comprising
(a) isolating single cells from a tumor tissue extracted from the human body;
(b) removing CD25⁺ cells from the single cells obtained in step (a) and then culturing the resulting cells in the presence of one or more cytokines selected from the group consisting of interleukin-2, interleukin-15, and interleukin-21; and
(c) culturing the cells obtained in step (b) in the presence of one or more cytokines selected from the group consisting of interleukin-2, interleukin-15, and interleukin-21 and peripheral blood mononuclear cells.

2. The method according to claim 1, wherein the cytokine in step (b) is interleukin-2.

3. The method according to claim 2, wherein the interleukin-2 in step (b) is present in an amount of 10 to 6,000 U/ml.

4. The method according to claim 1, wherein the cytokine in step (b) is a mixture of interleukin-2, interleukin-15, and interleukin-21.

5. The method according to claim 4, wherein the interleukin-2, interleukin-15, and interleukin-21 in step (b) are present in an amount of 10 to 6,000 U/ml, 20 to 300 U/ml, and 5 to 100 U/ml, respectively.

6. The method according to claim 1, wherein the culturing in step (b) is carried out for 5 to 60 days.

7. The method according to claim 1, wherein the culturing in step (b) is carried out further in the presence of an anti-CD3/28 antibody.

8. The method according to claim 7, wherein the anti-CD3/28 antibody is present in an amount of 5 to 50 µg/ml.

9. The method according to claim 7, wherein the culturing in step (b) is carried out by culturing in the presence of the cytokine and then culturing while replacing 1/3 to 2/3 of the medium with a medium comprising the anti-CD3/28 antibody and the cytokine every 4 to 6 days.

10. The method according to claim 9, wherein the culturing is carried out while adding the cytokine or a medium containing the cytokine every 2 to 3 days between the medium replacements every 4 to 6 days.

11. The method according to claim 1, wherein the culturing in step (c) is carried out further in the presence of phytohemagglutinin.

12. The method according to claim 11, wherein the phytohemagglutinin is present in an amount of 1 to 20 µg/ml.

13. The method according to claim 1, wherein the culturing in step (c) is carried out by culturing in the presence of the cytokine, peripheral blood mononuclear cells, and optionally phytohemagglutinin and then culturing while supplementing the cytokine every 2 to 3 days from the 5th day.

14. A method for preparing tumor-infiltrating lymphocytes, comprising
(a') isolating single cells from a tumor tissue extracted from the human body;
(b') culturing the single cells obtained in step (a') in the presence of an anti-GITR antibody and one or more cytokines selected from the group consisting of interleukin-2, interleukin-15, and interleukin-21; and
(c') culturing the cells obtained in step (b') in the presence of one or more cytokines selected from the group consisting of interleukin-2, interleukin-15, and interleukin-21 and peripheral blood mononuclear cells.

15. The method according to claim 14, wherein the anti-GITR antibody is present in an amount of 1 to 100 µg/ml.

16. The method according to claim 14, wherein the cytokine in step (b') is interleukin-2.

17. The method according to claim 16, wherein the interleukin-2 in step (b') is present in an amount of 10 to 6,000 U/ml.

18. The method according to claim 14, wherein the cytokine in step (b') is a mixture of interleukin-2, interleukin-15, and interleukin-21.

19. The method according to claim 18, wherein the interleukin-2, interleukin-15, and interleukin-21 in step (b') are present in an amount of 10 to 6,000 U/ml, 20 to 300 U/ml, and 5 to 100 U/ml, respectively.

20. The method according to claim 14, wherein the culturing in step (b') is carried out for 5 to 60 days.

21. The method according to claim 14, wherein the culturing in step (b') is carried out further in the presence of an anti-CD3/28 antibody.

22. The method according to claim 21, wherein the anti-CD3/28 antibody is present in an amount of 5 to 50 µg/ml.

23. The method according to claim 21, wherein the culturing in step (b') is carried out by culturing in the presence of the cytokine and the anti-GITR antibody and then culturing while replacing 1/3 to 2/3 of the medium with a medium comprising the anti-CD3/28 antibody and the cytokine every 4 to 6 days.

24. The method according to claim 23, wherein the culturing is carried out while adding the cytokine or a medium containing the cytokine every 2 to 3 days between the medium replacements every 4 to 6 days.

25. The method according to claim 14, wherein the culturing in step (c') is carried out further in the presence of phytohemagglutinin.

26. The method according to claim 25, wherein the phytohemagglutinin is present in an amount of 1 to 20 µg/ml.

27. The method according to claim 14, wherein the culturing in step (c') is carried out by culturing in the presence of the cytokine, peripheral blood mononuclear cells, and optionally phytohemagglutinin and then culturing while supplementing the cytokine every 2 to 3 days from the 5th day.
